(19)

**Europäisches Patentamt**

**European Patent Office**

**Office européen des brevets**

(11) **EP 4 252 770 A1**

(12) **EUROPEAN PATENT APPLICATION**

(43) Date of publication:
04.10.2023 Bulletin 2023/40

(21) Application number: 22166276.0

(22) Date of filing: 01.04.2022

(51) International Patent Classification (IPC):
**A61K 39/108** *(2006.01)* **A61P 31/04** *(2006.01)*

(52) Cooperative Patent Classification (CPC):
**A61K 39/0258; A61P 31/04;** A61K 2039/521;
A61K 2039/552

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Veterinärmedizinische Universität
Wien
1210 Vienna (AT)**

(72) Inventors:
• **HESS, Michael
3400 Klosterneuburg (AT)**
• **PAUDEL, Surya
1030 Wien (AT)**
• **HESS, Claudia
3400 Klosterneuburg (AT)**

(74) Representative: **SONN Patentanwälte OG
Riemergasse 14
1010 Wien (AT)**

(54) **COMPOSITION COMPRISING IRRADIATED E. COLI**

(57) Disclosed is a composition comprising an immunologically effective amount of cells of *Escherichia coli* (*E. coli*) species killed by irradiation in a pharmaceutically acceptable carrier, wherein said killed cells are produced by exposing whole, intact cells of an *E. coli* species to irradiation under conditions effective to kill said cells so as to obtain a complete loss of multiplication ability in the cells while retaining the ability to elicit a protective immune response in an animal against said *E. coli* species, wherein the ability is characterised by a metabolic activity of the composition comprising the irradiated E. coli species of at least 25 %, preferably at least 35 %, especially at least 50 %, in a resazurin-based test for metabolic activity, wherein metabolic activity is measured by the percentage of reduction of resazurin (7-hydroxy-3H-phenoxazin-3-one 10-oxide) to resorufin (7-hydroxy-3H-phenoxazin-3-one).

EP 4 252 770 A1

**Description**

The present invention relates to a novel vaccine against *Escherichia coli* (*E. coli*), which offers superior protection and safety over existing vaccines.

*Escherichia coli,* a member of the family Enterobacteriaceae, has inconsistent or disputed reputation with regard to its pathogenicity because it resides in the intestine in symbiosis with its host as one of the major components of the gut microflora and is also acknowledged to be responsible for several extraintestinal diseases summarized as colibacillosis in all age groups of chickens (Nolan et al., 2020). The most common lesions during the bacterial systemic infection in broilers and layers are aerosaculitis, perihepatitis, pericarditis and/or salpingitis/peritonitis. Colibacillosis leads to very high economic losses mainly due to mortality or decrease in performance and carcass condemnation. Antibiotic resistance in *E. coli* has been reported in surveillance data and in scientific studies from all major poultry producing countries around the world (Roth et al., 2019). Strikingly, the resistance of *E. coli* against most antimicrobials has clearly risen over the past two decades (Nhung et al., 2017). Furthermore, close similarities of avian isolates to human uropathogenic *E. coli* in terms of genotypic traits strongly suggest that some isolates have zoonotic potential as well (Johnson et al., 2007; Rodriguez-Siek et al., 2005).

For prevention of the disease, numerous efforts have been made in the past to develop an effective vaccine but most have been only limited to the laboratory trials (Ghunaim et al., 2014; Swelum et al., 2021). Second-generation vaccine candidates such as subunit vaccines induce less broad reacting antibodies, which are altogether of limited value due to high heterogeneity and the complexity of genetic traits of *E. coli*. Traditional use of chemicals such as formaldehyde for inactivation of whole bacterial cells is very harsh and might induce impaired antigenic structures. Among various methods for preparation of killed vaccines, ultrasonication was found to be the most effective for inactivation as compared to irradiation (as suggested for *Salmonella* species in US 8,173,139 B1; yet without a notable scientific or industrial impact or proof for efficiency, formalin or heat treatment when birds were vaccinated subcutaneously (Melamed et al., 1991). In recent years, autogenous vaccines are also being used but the limited published studies demonstrated that the vaccine was beneficial to protect against homologous but not heterologous challenge (Landman and van Eck, 2017). In another study no protection at all was noticed in progenies obtained from breeders following single or double vaccination ; Li et al., 2017).

Three commercial vaccines against *E. coli* infection are currently licensed. An inactivated *E. coli* vaccine is given intramuscular for passive protection of progeny through vaccinated broiler breeders (Nobilis® E. coli Inac., MSD Animal health). In a field study using this vaccine, lower incidences of colibacillosis were observed in vaccinated breeders but there were no differences in early week mortality, average body weight and feed conversion ratio of broilers originating from vaccinated or non-vaccinated parents (Gregersen et al., 2010). An *aro*A deletion mutant of *E. coli* (serogroup O78) is licensed as a live vaccine (Poulvac® E. coli, Zoetis), which is recommended to be administered via coarse spray (particle size > 100 $\mu$m) in day-old chickens followed by booster in drinking water (WO 2004/103402 A1). The vaccine was demonstrated to be protective against homologous or heterologous challenges (La Ragione et al., 2013). In contrast, in an earlier study, spray vaccination of birds with an *aro*A deletion mutant of another *E. coli* strain, also belonging to the O78 serogroup, was not protective against *E. coli*-O2 challenge, thus the protection of the mutant was concluded to be serogroup specific (Kariyawasam et al., 2004). Furthermore, in two separate studies in Egypt, the Poulvac® vaccine was not able to protect birds against experimental challenge with *E. coli*-O125 or *E. coli*-O1 (Gharib et al., 2017; Mohamed et al., 2016) . In a recently published study it was noticed that the Poulvac® vaccine showed higher efficacy when given prior to an autogenous vaccine compared to its sole use (Koutsianos et al., Vet. Sci. 7 (2020), 80). A second live attenuated vaccine is marketed in Japan and was developed by deletion of the *crp* gene of an *E. coli*-O78 strain. The vaccine is delivered via fine spray (20 $\mu$m droplet size) and was shown to be effective only against the homologous challenge but not against a heterologous challenge with *E. coli*-O125 (Abd El-Mawgoud et al., 2020). Thus, expanding the portfolio of *E. coli* vaccine in chickens is a high priority in poultry industry.

From immunological point of view, a recent review highlighted the influence of *E. coli* infection in chickens on mucosal immunity inducing a strong cellular innate immune response (Alber et al., 2021). Likewise, administration of the aroA-deleted mutant strain (Poulvac® vaccine) in broilers was shown to increase cellular immune response, mainly CD4$^+$TCRV$\beta$1$^+$ cells that are related to the respiratory mucous membrane, and the protection was not correlating with the circulating antibodies (Filho et al., 2013). In order to enhance the mucosal immunity, the vaccine particles should come in contact with the maximum surface area of the upper as well as lower respiratory organs (Hellfritzsch and Scherließ, 2019). In young chickens the efficiency of inhalation of particles > 20$\mu$m to reach to lungs is very low (Corbanie et al., 2006) . Considering all these facts, the recommended application methods for the currently available live attenuated vaccines of *E. coli* may not be ideal to allow deposition of vaccine particles into lower respiratory organs.

It is an objective of the present invention to provide means for controlling respiratory and/or enteric *E. coli* bacteria in animals, and particularly for preventing colibacillosis and their symptoms, such as aerosaculitis, perihepatitis, pericarditis and/or peritonitis, in poultry, especially in young chickens. It is a further objective of the present invention to provide an economically feasible vaccination concept to efficiently prevent colibacillosis. It is desirable to provide such vaccines without applying chemicals, such as formaldehyde. Moreover, such means should be easily deliverable, preferably without the need for injecting such vaccines to the animals, at commercially viable conditions. Therefore, the present invention provides a composition comprising an immunologically effective amount of cells of *Escherichia coli* (*E. coli*) species killed by irradiation in a pharmaceutically acceptable carrier, wherein said killed cells are produced by exposing whole, intact cells of an *E. coli* species to irradiation under conditions effective to kill said cells so as to obtain a complete loss of multiplication ability in the cells while retaining the ability to elicit a protective immune response in an animal against said *E. coli* species, wherein the ability is characterised by a metabolic activity of the composition comprising the irradiated *E. coli* species of at least 25 %, preferably at least 35 %, especially at least 50 %, in a resazurin-based test for metabolic activity, wherein metabolic activity is measured by the percentage of reduction of resazurin (7-hydroxy-3H-phenoxazin-3-one 10-oxide) to resorufin (7-hydroxy-3H-phenoxazin-3-one).

With the present invention, effective means for controlling respiratory and/or enteric *E. coli* bacteria in animals, are provided which are efficiently preventing colibacillosis and their symptoms, such as aerosaculitis, perihepatitis, pericarditis and/or peritonitis. The present composition is specifically suitable in poultry, especially in young chickens, because it allows an economically feasible instrument for bacterial control in industrial production. The present invention is specifically suitable due to its efficient cross-reactivity management which allows also protection against colibacilloses caused by non-homologous and/or antibiotic-resistant bacterial strains. Another advantage of the present invention is that the bacterial antigens can be produced without applying chemicals, such as formaldehyde, therefore reducing the chemical load or preventing production steps eliminating such chemicals. The present composition can effectively be applied as an aerosol without the need for work-intensive administration steps, such as vaccine injections or other peritoneal administrations needing administration devices other than simple devices, such as aerosol delivering devices.

Remarkably and unexpectedly, with the present invention a clear correlation between the (remaining) metabolic activity of irradiated cells and the appropriateness of the compositions comprising the killed cells as vaccines was observed and is used as a paramount feature of the compositions according to the present invention. It could be shown with the present invention that the higher the metabolic activity was preserved (in absolute % terms) after irradiation, the more suitable the composition turned out to be in its performance as vaccines, especially in the ability to induce protection (homologous and heterologous) *in vivo*.

Moreover, it was shown with the present invention that even an amount of 25 % metabolic activity was already suitable to gain at least a certain homologous protection (e.g. with strain PA15/19103-3), the higher metabolic activity was preserved after irradiation (especially in strains which already have a high "baseline" metabolic activity (i.e. before irradiation), the better was the *in vivo* protection obtained (such as the good homologous and heterologous protection provided with strain PA14/17480/5).

It is essential for the present method that the cells of the composition are killed by the irradiation so that they do not replicate/multiplicate anymore. This loss of multiplication ability is important to safeguard that the composition does not elicit pathological risks for the animals to be vaccinated. Loss of replication/multiplication ability may be verified by lack of growth of the irradiated cells on agar plates where viable cells replicate/multiply, for example by showing no growth after streaking the irradiated cells on MacConkey agar plates and cultivating the plates at 37°C for at least 24h. On the other hand, irradiation has to be performed in a way so as to retain the ability to elicit a protective immune response in an animal against the *E. coli* type. This requires care not to completely destroy the cells in their native form and presentation of antigens on the surface of the irradiated cells.

*E. coli* is a Gram-negative, facultative anaerobic, rod-shaped, coliform bacterium. *E. coli* cells are typically rod-shaped, and are 1 to 6 $\mu$m (typically about 2.0 $\mu$m) long and 0.25 - 1.0 $\mu$m in diameter, with a cell volume of about 0.6-0.7 $\mu$m$^3$.

According to a preferred embodiment of the present invention, retaining the ability to elicit a protective immune response is safeguarded (as a surrogate) by the presence of metabolic activity of the irradiated cells (despite their loss of multiplication ability). Presence of (residual) metabolic activity despite killing the cells (with respect to their ability to replicate) shows that the irradiation was sufficient for eliminating the multiplication ability (required for safety of the vaccine) but soft enough to keep the antigens presented on the bacterial outer surface in a native manner so as to elicit an appropriate immune response in an animal which is vaccinated with the irradiated cells (required for efficacy).

The presence of metabolic activity can be tested by a variety of established test systems, for example the resazurin test system. Resazurin (7-Hydroxy-3H-phenoxazin-3-one 10-oxide) is a phenoxazine dye that is weakly fluorescent, nontoxic, cell-permeable, and redox-sensitive. Resazurin has a blue to purple colour (at pH > 6.5) and may be used in microbiological, cellular, and enzymatic assays because it can be irreversibly reduced to the pink-coloured and highly fluorescent resorufin (7-Hydroxy-3H-phenoxazin-3-one). At circum-neutral pH, resorufin can be detected by visual observation of its pink colour or by fluorimetry, with an excitation maximum at 530-570 nm and an emission maximum at 580-590 nm (Haggerty et al., J. Geophys. Res. 114 (2009), 942; Costa et al., Antibiotics 19 (2021), 974; , UptiBlue viable cell assay, technical manual). Commercial test available are e.g. the alamarBlue™ cell viability assay (Invitrogen, Austria), or AB assay (Trek Diagnostic Systems, Inc.), Vybrant (Molecular Probes) and UptiBlue (In-terchim). Accordingly, metabolic activity is preferably present as verifiable by resazurin reduction to resorufin by aerobic respiration.

As also outlined in the example section, a preferred way how to establish appropriate metabolic activity of the killed cells used in the compositions according to the present invention is the well-known resazurin-based metabolic activity test. The metabolic activity (in %) can be determined according to the preferred method by using about $1 \times 10^8$ killed cells in a volume of 100 $\mu$l and incubating the cells and resazurin in a concentration of from 40 to 50 $\mu$M at a pH of from 7.0 to 7.5 for three hours at 37°, and then measuring optical densities (ODs), preferably with an enzyme linked immunosorbent assay (ELISA) reader, at wavelengths of 600 and 550 nm and determining the percentage reduction of resazurin according to the following equation:

$$\text{Percentage reduction of resazurin} = [A_{LW} - (A_{HW} \times R_O)] \times 100;$$

$A_{LW}$ = absorbance at lower wavelength minus the media blank
$A_{HW}$ = absorbance at higher wavelength minus the media blank
$R_O$ = correction factor (0.50466).

According to a preferred embodiment, the composition according to the present invention is an aerosol composition, i.e. a composition suitable for aerosol delivery. According to this preferred embodiment, the composition comprises the cells as aerosol particles with an average diameter of 20 $\mu$m or lower, preferably of 1 to 20 $\mu$m, especially of 2 to 10 $\mu$m. A preferred method for determination of average diameters in aerosols is the laser diffraction method (ISO 9276-2:2014, ISO 13320:2009).

In a preferred embodiment, the composition further comprises an adjuvant, preferably Freund's incomplete adjuvant, Freund's complete adjuvant, alum, microparticles, nanoparticles, beads, and oil. The phrase "pharmaceutically acceptable carrier" as used herein means a pharmaceutically acceptable material, composition or vehicle, such as a liquid or solid filler, diluent, excipient, or solvent encapsulating material known and applied in pharmaceutical practice, especially in veterinary practice.

Preferably, killed cells according to the present invention are produced by irradiation. A variety of conventional high or low energy linear accelerators are suitable for generating the E-Beam used herein, although linear accelerators having beam energies above approximately 1.0 MeV are preferred. However, it is envisioned that other suitable electron beam accelerators include electrostatic direct-current (DC), electrodynamic DC, linear accelerators (LINACS), magnetic-induction LINACs, and continuous-wave (CW) accelerators.

For practical purposes, the cells are preferably prepared by suspension in a liquid, for example, the culture medium or a diluent such as physiologically buffered saline, for irradiation. In a preferred embodiment, the suspension is prepared/adjusted to provide an approximate concentration between $10^6$ to $10^{11}$, preferably of $10^6$ to $10^{10}$, preferably of $10^7$ to $10^9$ CFU/ml.

An effective irradiation treatment for killing the cells is defined herein as that which kills 99.99999% or more of the viable cells, without destroying the cell surface properties or lysing the cells but still retaining the ability of the cells to elicit an antibody response in the animal. While treatments killing 100% of all viable cells would typically be preferred, the skilled practitioner will recognize 100% cell death may not always be readily obtainable.

Therefore, the preferred and proper test for identifying a complete loss of multiplication ability of the killed cells according to the present invention is determined by:

(i) streaking suspensions of the irradiated cells on MacConkey agar plates and enrich aliquots in LB (Luria-Bertani) broth in parallel,
(ii) incubating both, agar plates and broth at 37°C for overnight,
(iii) streaking the culture in LB broth the next day on MacConkey agar plates and incubating agar plates at 37°C for overnight, and
(iv) detecting no growth on agar plates before and after in vitro enrichment.

This test system establishes - on the basis of well-known procedures and media (LB) a reliable and reproducible test system. With this test, a significant reliability is provided to ascertain that the cells according to the present invention are practically free of having any relevant danger to the avian recipients of the present compositions.

The irradiation dose to effect this killing may vary somewhat, but should not be so great as to disrupt (lyse) the cells or significantly alter or destroy cell-surface antigens, and may be readily determined by routine testing. However, as a practical matter, the cells should preferably receive an irradiation dosage sufficient to arrive at the above defined inactivation test threshold.

The radiation dose necessary to achieve efficient killing of cells will of course vary with the optical density of the cell culture or suspension, the genetics of the particular *E. coli* species or strain, the physical state of the culture (lyophilized, in suspension, or frozen), the material composition of the sample container and packaging, the electron beam current (measured in milliamperes), and the path length of the medium containing the cells (i.e., the depth through which the radiation must pass) and should be selected to provide the desired radiation dose. The temperature of the treatment also is not critical, although very high temperatures inducing protein denaturization should be obviously avoided. The process temperature should be between about 1 to 10° C to avoid thermal inactivation of the organisms.

In fact, the irradiation may be individually "titrated" to provide the appropriate or chosen metabolic activity. Accordingly, it is possible by selection of strains with a given metabolic activity to apply irradiation to the extent needed for complete loss of viability (in terms of multiplication ability; i.e. resulting in a complete loss of multiplication ability) but (only) to the extent needed to preserve the desired metabolic activity. In applying the teachings of the present invention, the metabolic activity of the killed cells in the composition can preferably be kept at least 60 %, more preferred at least 70 %, especially at least 80 %, in the resazurin-based test for metabolic activity.

The composition according to the present invention preferably comprises a dose of killed cells of $10^6$ to $10^{11}$ cells, preferably of $10^7$ to $10^{10}$ cells, especially of $1,0 \times 10^8$ to $1.5 \times 10^9$ cells.

Preferably, killed cells in the composition of the present invention are PA14/17480/5-ovary, PA15/19103-3, or mixtures thereof, preferably PA14/17480/5-ovary (Rezaee et al., Vet. Pathol. 58 (2021), 71-79; Gaußmann et al., Avian Pathol. 47 (2018), 271-280) .

According to a preferred embodiment of the present invention the cells are cells of an avian pathogenic *E. coli* (APEC) strain or mixtures of APEC strains. APEC strains are well known in the present field of technology and e.g. referred to in Bagheri et al. (Frontiers (2022), 864656), Alber et al., Kathayat et al., Pathogens 10 (2021), 0046; Mehat et al., Av. Pathol. 50 (2021), 402-416; Thomrongsuwannakij et al., Poultry Sci. 101 (2022), 101527; Newman et al., PeerJ 9 (20121), e1102, and many more.

According to another aspect, the present invention relates to a composition according to the present invention for use in a method for controlling an *E. coli* species in an animal comprising administering to the animal the composition to elicit a protective immune response in an animal against said *E. coli* species. Therefore, the present invention is also drawn to a method for vaccinating animals with an effective amount of the composition of the present invention and/or a method for controlling an *E. coli* species in an animal comprising administering to the animal the composition to elicit a protective immune response in an animal against said *E. coli* species. Accordingly, the present invention also relates to the use of a composition according to the present invention for the manufacture of a medicament for vaccinating an animal and/or for controlling an *E. coli* species in an animal comprising administering to the animal the composition to elicit a protective immune response in an animal against said *E. coli* species.

Preferably, the animal is poultry selected from the group consisting of chickens, turkeys, ducks, quail, and geese, preferably wherein the animal is an animal being younger than four weeks, especially younger than two weeks. However, also older animals may be subject to the present invention.

Preferably, the composition is administered as an aerosol to the animal.

The present invention is specifically suited for use in chicken. According to this preferred embodiment, the animal is chicken with an age of not more than 10 days, preferably with an age of not more than 7 days, especially of not more than 4 days.

The present invention is further described by the following examples and the figures, yet without being limited thereto.

**Figure 1.** Long term monitoring of percentage reduction of alamarBlue in three separate experiments. Suspensions were stored at 4°C. Values are expressed as mean±SEM. Different letters denote for statistically significant differences, $p \leq 0.05$.

**Figure 2.** Transmission electron microscopy. A: non-irradiated process control *E. coli* PA14/ PA14/17480/5-ovary; B: the same strain irradiated with Gamma radiation; scale bar $1 \mu$m.

**Figure 3.** Protein profiling measured with Matrix Assisted Laser Desorption/Ionization-Time Of Flight-Mass Spectrometry. Spectra of non-irradiated process control *E. coli* PA14/ PA14/17480/5-ovary (red) and the same strain irradiated with Gamma radiation (black).

**Figure 4.** Macroscopic lesions. A: aerosaculitis, group 2, at 3 days post infection (dpi), trial 1; B: fibrinous perihepatitis, group 2 at 13 dpi, trial 1; C: the same bird, pericarditis; D: perihepatitis and pericarditis, group 4 at 10 days post challenge (dpc), trial 2; E: cloudy air sac and slight peritonitis, group 4 at 10 dpc, trial 2.

**Figure 5.** Emean values of histopathology lesion scores in birds of animal trial 1 at 3 days post infection/vaccination; groups 1 (vaccinated with irradiated cells), 2+3 (infected with live *E. coli*), 4 (vaccinated with formalin killed cells) and 5+6 (not vaccinated/infected).

**Figure 6.** Emean values of histopathology lesion scores in birds of animal trial 1 after challenge, values of all birds sequentially killed after challenge were analysed together; groups 1 (vaccinated with irradiated cells and challenged), 2(infected with live *E. coli* and challenged), 4 (vaccinated with formalin killed cells and challenged) and 5 (not vaccinated but challenged) .

**Figure 7.** Emean values of histopathology lesion scores in birds of animal trial 2 after challenge (comparison of routes of vaccination). All birds sequentially killed after challenge were combined together; groups 1 (vaccinated with irradiated cells via aerosol and homologous challenged), 2(vaccinated with irradiated cells via oculonasal and homologous challenged), 4 (not vaccinated but challenged).

**Figure 8.** Emean values of histopathology lesion scores in birds of animal trial 2 after challenge (comparison of homologous and heterologous protection). All birds sequentially killed after challenge were combined together; groups 1 (vaccinated with irradiated cells via aerosol and homologous challenged), 3 (vaccinated with irradiated cells via aerosol and heterologous challenged), 4 (not vaccinated but homologous challenged), 5 (not vaccinated but heterologous challenged).

**Figure 9.** Pattern of *E. coli* reisolation from different organs of birds after challenge in animal trial 2 (comparison of routes of vaccination). All birds sequentially killed after challenge were combined together; groups 1 (vaccinated with irradiated cells via aerosol and homologous challenged), 2 (vaccinated with irradiated cells via oculonasal and homologous challenged), 4 (not vaccinated but challenged).

**Figure 10.** Pattern of *E. coli* reisolation from different organs of birds after challenge in animal trial 2 (comparison of homologous and heterologous protection). All birds sequentially killed after challenge were combined together; groups 1 (vaccinated with irradiated cells via aerosol and homologous challenged), 3 (vaccinated with irradiated cells via aerosol and heterologous challenged), 4 (not vaccinated but homologous challenged), 5 (not vaccinated but heterologous challenged).

**Figure 11.** Antibody titre measured against PA14/17480/5-ovary in animal trial 1. For trial design refer to Table 1. Different letters at the same time point denote for statistically significant differences.

**Figure 12.** Antibody titre measured against PA14/17480/5-ovary or PA16/13200-animal2/clone 3 in animal trial 2. For trial design refer to Table 2. Plates used for groups 1, 2, 4 and 6 were coated with PA14/17480/5-ovary (homologous strain) and those for groups 3 and 5 were coated with PA16/13200-animal2/clone 3 (heterologous strain). Different letters at the same time point denote for statistically significant differences.

**Figure 13.** Histopathology in animal trial 3: Group 1: PA14/17480/5-ovary; group 2: PA 15/19103-3, group 3: 15/25396-3 right, group 4: 15/24960-2, group 5: negative control.

**Figure 14.** Determination of percentage reduction of alamarBlue by two E. coli isolates of different serotypes in animal trial 4.

**Figure 15 A.** Antigen: PA14/17480/5-ovary (O1:K1) in animal trial 4:
Group 1: vaccinated with O1:K1 and challenged with O1:K1; group 2: vaccinated with O1:K1 and challenged with O78:K80; group 3: vaccinated with O78:K80 and challenged with O78:K80; group 4: positive control O1:K1 challenge (positive) control; group 5: challenge (positive) control O78:K80; group 6: negative control; groups with different superscripts are statistically different

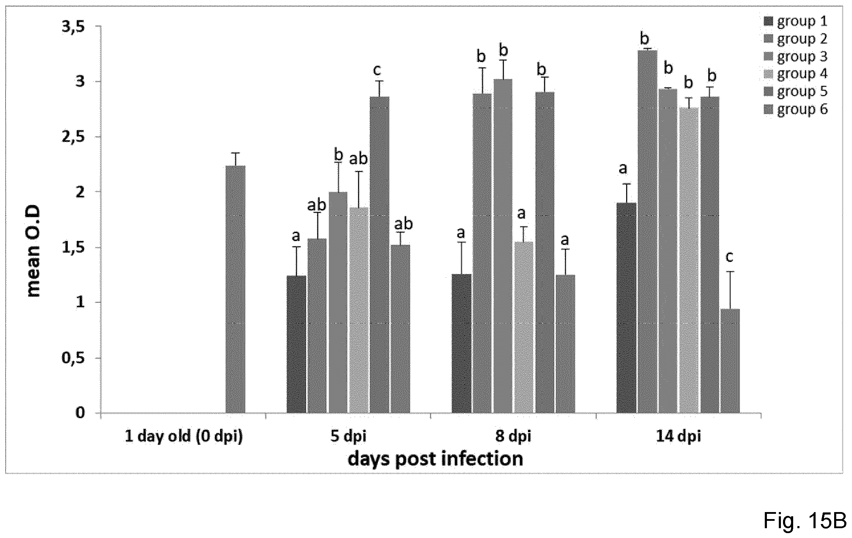

**Figure 15 B.** Antigen: 15/19103-3 (O78:K80) in animal trial 4:
Group 1: vaccinated with O1:K1 and challenged with O1:K1; group 2: vaccinated with O1:K1 and challenged with O78:K80; group 3: vaccinated with O78:K80 and challenged with O78:K80; group 4: positive control O1:K1 challenge (positive) control; group 5: challenge (positive) control O78:K80; group6: negative control; groups with different superscripts are statistically different

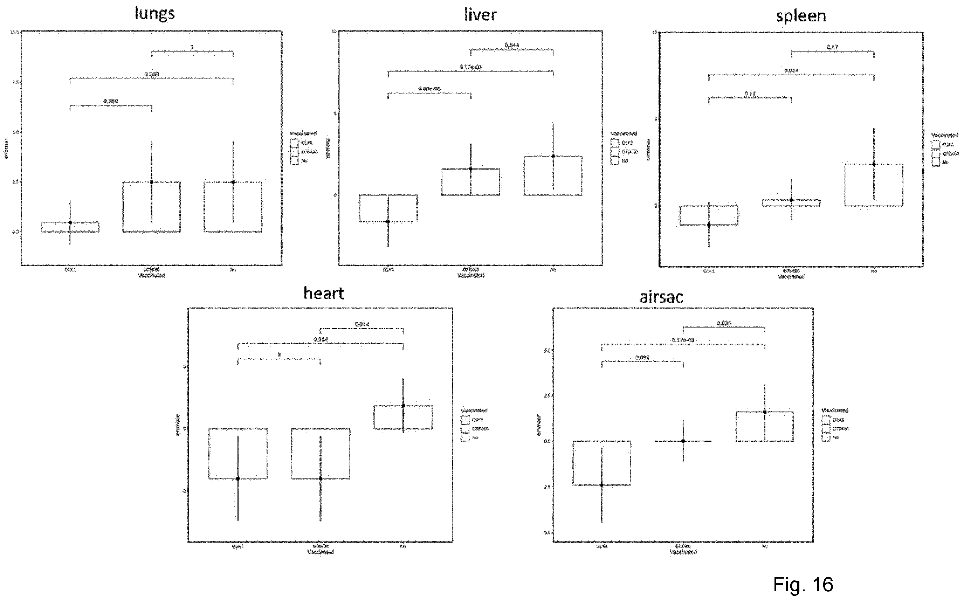

**Figure 16.** Pattern of bacterial re-isolation from different organs of birds of groups 2,3 and 5 in animal trial 4. Groups 2 and 3 were vaccinated with irradiated PA14/17480/5-ovary (O1:K1) or irradiated PA15/19103-3 (O78:K80) and group 3 was left without vaccination. All birds were challenged with PA15/19103-3.

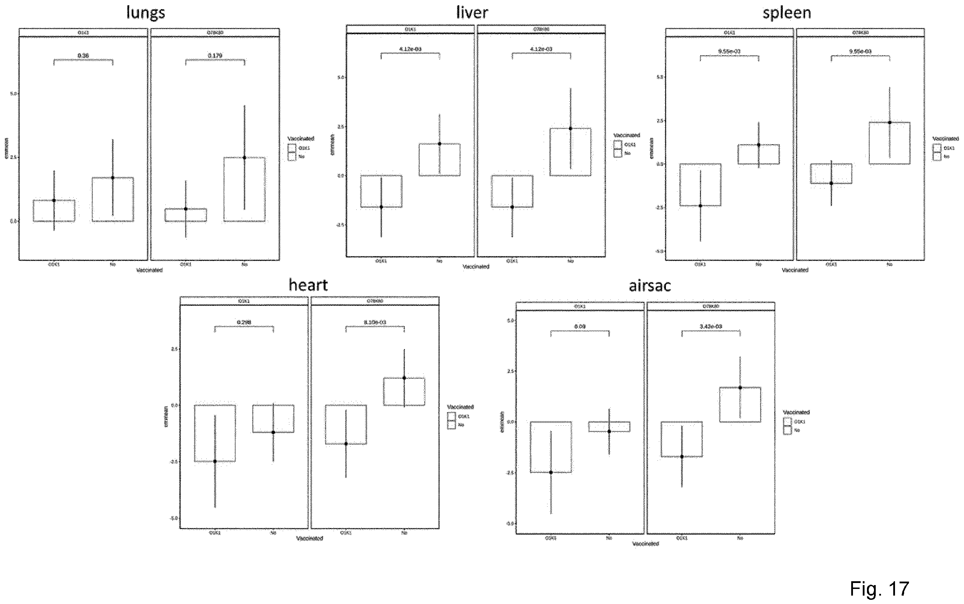

**Figure 17.** Pattern of bacterial re-isolation in birds of groups 1, 2, 4 and 5 in animal trial 4. Groups 1 and 2 were vaccinated with irradiated PA14/17480/5-ovary and challenged with PA14/17480/5-ovary (O1:K1) or PA15/19103-3 (O78:K80), respectively. Groups 4 and 5 were left without vaccination and challenged PA14/17480/5-ovary or PA15/19103-3, respectively

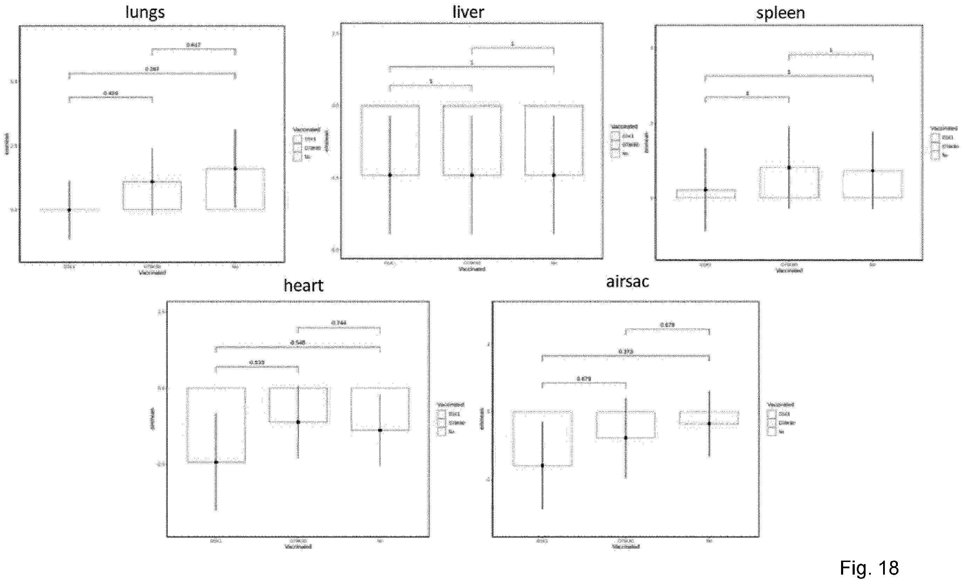

**Figure 18.** Pattern of histology lesion scores in different organs of birds of groups 2,3 and 5 in animal trial 4. Groups 2 and 3 were vaccinated with irradiated PA14/17480/5-ovary (O1:K1) or irradiated PA15/19103-3 (O78:K80) and group 3 was left without vaccination. All birds were challenged with PA15/19103-3.

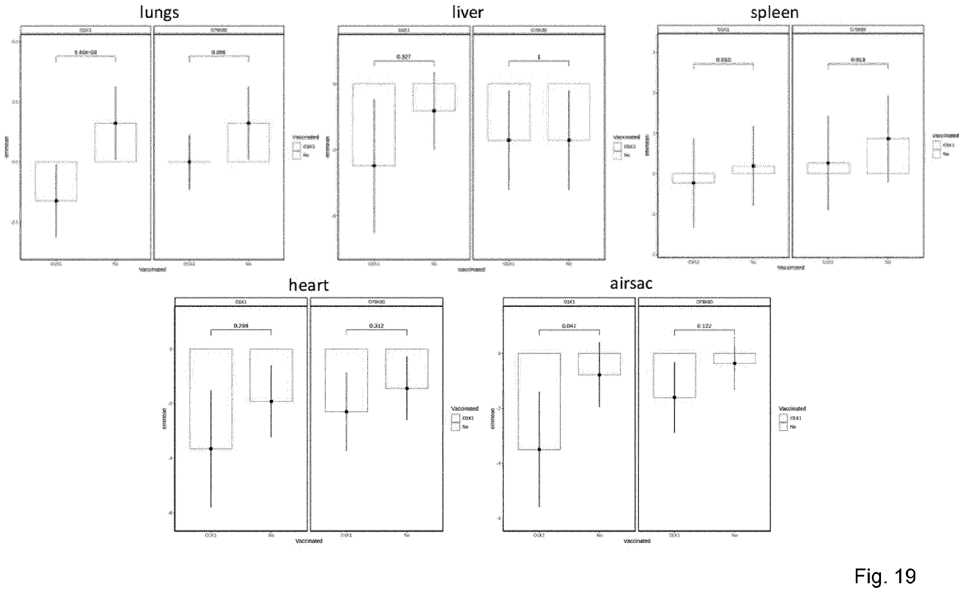

**Figure 19.** Pattern of histology lesion scores in birds of groups 1, 2, 4 and 5 in animal trial 4. Groups 1 and 2 were vaccinated with irradiated PA14/17480/5-ovary and challenged with PA14/17480/5-ovary (O1:K1) or PA15/19103-3 (O78:K80), respectively. Groups 4 and 5 were left without vaccination and challenged PA14/17480/5-ovary or PA15/19103-3, respectively.

Examples:

In the following examples, it was aimed to evaluate the protective efficacy of irradiated *E. coli* delivered via aerosol route (particle size 5 $\mu$m) in chickens. The use of ionizing radiation has a unique advantage to retain functional antigenic expression in bacterial cells keeping them metabolically active but inhibit their multiplication. Findings from *in vitro* experiments, two animal trials and associated laboratory investigations are described.

**Materials and methods**

**Bacterial isolates**

*E. coli* PA14/17480/5-ovary (serotype O1:K1) was used in all the experiments for the vaccine preparation. The strain was isolated from ovary of a diseased chicken with colibacillosis (Rezaee et al., 2020). Birds in animal trial 1 were challenged with *lux*-tagged *E. coli* PA14/17480/5-ovary. The bioluminescence locus *lux-ABCDE* was inserted into the chromosome of *E. coli* PA14/17480/5-ovary in order to be able to differentiate between the challenge strain and native *E. coli* isolates in chickens (Abdelhamid et al., 2020). In trial 2, challenge was done either with native *E. coli* PA14/17480/5-ovary (homologous challenge) or with *E. coli* PA16/13200-animal2/clone3 (heterologous challenge). The isolate *E. coli* PA16/13200-animal2/clone3 was obtained from the liver of a layer chicken affected with colibacillosis and could not be sero-typed using antibodies against O1:K1, O2:K1 or O78:K80 thus was assigned as non-typeable.

**Preparation of the irradiated vaccine**

The culture conditions and procedure for the preparation of suspensions for irradiation remained constant in all *in vitro* and *in vivo* experiments, which always yielded 8 log CFU/ml concentration of bacteria. *E. coli* PA14/17480/5-ovary was grown in LB broth at 37°C for six hours. Bacterial cells were harvested, washed two times and re-suspended in phosphate buffer saline (PBS). Finally, equal volume of 1 M concentration of sterile trehalose solution was added and colony forming unit (CFU) counts were determined. Process control suspensions were always included that were prepared and transported in the same way but were not irradiated. For irradiation, Gamma irradiation was performed with 1.2 Kgy of Cobalt source at the facilities of International Atomic Energy Agency laboratories, Seibersdorf, Austria.

In order to ensure that the irradiated cells do not replicate *in vitro,* freshly prepared irradiated suspensions were streaked on MacConkey agar plates and aliquots were enriched in LB broth in parallel. Both agar plates and broth were incubated at 37°C for overnight. On the next day, the culture in broth was streaked on MacConkey agar plates. Loss of complete multiplication ability of irradiated bacterial cells was verified after no growth were seen on agar plates before and after *in vitro* enrichment.

**Preparation of formalin-killed vaccine**

*E. coli* PA14/17480/5-ovary was cultivated in LB broth using the same protocol as mentioned above for irradiated vaccine except trehalose was not added after final resuspension of pellets. Cells were then treated with paraformaldehyde at a final concentration of 0.5% at room temperature for 24 hours and Freund's adjuvant was added at 1:1 ratio before vaccination.

**alamarBlue assay**

alamarBlue™ cell viability assay (Invitrogen, Austria) was performed to determine the metabolic activity in irradiated, formalin-killed and non-irradiated live cells (process control) cells according to the manufacturer's instruction. Briefly, 90 $\mu$l of test suspensions were mixed with 10 $\mu$l of alamarBlue (alamarBlue cell viability reagent, Invitrogen, Austria) in triplicates in each well of 96 well plates. After 3 hours of incubation at 37°C, optical densities (OD) were measured with an Enzyme Linked Immuno-sorbent Assay (ELISA) reader at wavelengths of 600 and 550 nm. Percentage reduction of alamarBlue was calculated according to the following equation:

$$\text{Percentage reduction of alamarBlue} = [A_{LW} - (A_{HW} \times R_O)] \times 100$$

$A_{LW}$ =absorbance at lower wavelength minus the media blank
$A_{HW}$ =absorbance at higher wavelength minus the media blank
$R_O$ = correction factor (0.50466)

**Electron microscopy**

The transmission electron microscopy was performed at the Electron Microscopy Facility, Vienna BioCenter Core Facilities (VBCF), Vienna, Austria in order to compare the structural differences in irradiated and non-irradiated *E. coli* PA14/17480/5-ovary cells. For this, bacterial cells were adsorbed onto the carbon side of a 400mesh Cu/Pd grid (Agar Scientific, UK). Grids were previously coated with a 4nm continuous carbon support film (self-made in an Edwards 306 Auto high vacuum evaporator) and glow-discharged in a BAL-TEC SCD005 sputter coater (BAL-TEC, Liechtenstein).

After 1 min adsorption time, unbound bacteria were washed off with a drop of stain. Subsequently grids were contrasted for 1 min with 0.25 % phosphotungstic acid (Sigma-Aldrich, Germany) adjusted to pH7 with NaOH. Grids were inspected in an FEI Morgagni 268D transmission electron microscope (FEI, Eindhoven, the Netherlands) operated at 80 kV. Positions on the grid were chosen randomly. Digital images were acquired using an 11 megapixel Morada CCD camera from Olympus-SIS (Germany).

**MALDI-TOF mass spectrometry**

Matrix Assisted Laser Desorption/Ionization-Time Of Flight Mass Spectrometry (MALDI-TOF MS) was performed in order to analyze the protein profile spectra of freshly prepared irradiated and process control *E. coli* PA14/17480/5-ovary cells using Ultraflex II MALDI-TOF mass spectrometer (Bruker Daltonik GmBH, Leipzig, Germany) following a previously described protocol (Alispahic et al., 2010).

**Animal experiments**

Two animal trials were performed in the actual study and were approved by the institutional ethics and animal welfare committee and the national authority according to §§ 26ff. of Animal Experiments Act, Tierversuchsgesetz 2012-TVG 2012 (license Number GZ 68.205/0195-V/3b/2019, Amendment GZ 2020-0.380.498).

**Animal trial 1**

The first animal trial was conducted to evaluate the protectivity of birds following vaccination with irradiated, live or formalin-killed *E. coli* cells. As shown in Table 1, 96 specified pathogen-free (SPF) layer chickens (VALO Biomedica GmbH, Sachsenring, Germany) were hatched and divided into 6 groups. Each group of birds was placed in separate isolators with negative pressure. At 7 days of age, birds in group 1 were immunized with irradiated *E. coli* suspension (1 ml/bird, concentration before irradiation: $7.8 \times 10^8$ CFU/ml) whereas birds in groups 2 and 3 were infected with live *E. coli* PA14/17480/5-ovary (1 ml/bird, concentration $7.9 \times 10^8$ CFU/ml) via aerosol route. In group 4, each bird was injected intramuscular with 0.5 ml of formalin-killed bacterin mixed with adjuvant (concentration before treatment with formalin: $4.2 \times 10^8$ CFU/ml). Three days after the first vaccination/infection, 4 birds from each group were euthanized for necropsy and sampling. At three weeks of age, booster doses were given to birds in group 1 with irradiated cells via oculonasal route (0.5 ml/bird, concentration before irradiation: $3.1 \times 10^8$ CFU/ml) and birds in group 4 were injected intramuscular with formalin-killed *E. coli* mixed with adjuvant (0.5 ml/bird, concentration before treatment with formalin: $4 \times 10^8$ CFU/ml). At five weeks of age, birds in groups 1, 2, 4 and 5 were challenged with lux-tagged PA14 *E. coli* PA14/17480-5-ovary (1 ml/bird, concentration $2 \times 10^8$ CFU/ml), whereas birds in groups 3 and 6 were inoculated with the same volume of PBS via aerosol route. At 3, 7 and 10 days post challenge (dpc), birds from each group were sequentially euthanized for necropsy and sampling was performed. Clinical signs were recorded daily and blood samples were collected from all birds at weekly interval.

For aerosolization, birds were kept in a plastic box (approx. 55 L) inside isolators and airflows were switched off. Suspensions were nebulized inside the box with a small particle nebulizer (NebulAIR+, Flaem, Italy) that generates breathable fraction of droplets < 5 μm (81.5%).

Table 1: Experimental design of animal trial 1

| group | vaccination/ infection (7 days of age) [a] | necropsy/ sampling (10 days of age) | booster vaccination (21 days of age) | challenge (35 days of age)[b] | necropsy/sampling (days post challenge) | | |
|---|---|---|---|---|---|---|---|
| | | | | | 3 | 7 | 10 |
| 1 (n=16) | irradiated *E. coli* (aerosol) | 4[c] | irradiated *E. coli* (oculonasal) | yes | 4 | 4 | 4 |
| 2 (n=16) | live *E. coli* (aerosol) | 4 | no | yes | 3[d] | 4 | 4 |
| 3 (n=16) | live *E. coli* (aerosol) | 4 | no | no | 4 | 4 | 4 |
| 4 (n=16) | formalin-killed *E. coli* (IM)[e] | 4 | formalin-killed *E. coli* (IM) | yes | 4 | 4 | 4 |
| 5 (n=16) | no | 4 | no | yes | 4 | 4 | 4 |

(continued)

| group | vaccination/ infection (7 days of age) [a] | necropsy/ sampling (10 days of age) | booster vaccination (21 days of age) | challenge (35 days of age)[b] | necropsy/sampling (days post challenge) | | |
|---|---|---|---|---|---|---|---|
| | | | | | 3 | 7 | 10 |
| 6 (n=16) | no | 4 | no | no[d] | 4 | 4 | 4 |

[a]*E. coli* PA14/17480/5-ovary was used for immunization/infection at 7 and 21 days of age; [b]*lux*-tagged *E. coli* PA14/17480/5-ovary was used for aerosol challenge of birds at 35 days of age; controls obtained PBS; [c]number of birds killed for sampling; [d] one bird was euthanized before scheduled killing due to severe clinical signs; [e] IM: intramuscular.

**Animal trial 2**

The second animal trial was performed to investigate the suitability of the prototype vaccine application at early life stages, to compare efficacy of two different routes of vaccination and to evaluate protectivity against a heterologous challenge. Day-old SPF layer chickens (VALO Biomedica GmbH, Sachsenring, Germany) were divided into 6 groups with 12 birds in each (Table 2). At the first day of life, birds in groups 1 and 3 were immunized with irradiated *E. coli* cell suspensions via aerosol route (cell density before irradiation: $3.8 \times 10^8$ CFU/ml) and birds in group 2 were administered with the same suspension - volume and dose - via the oculonasal route. At two weeks of age, challenge was performed with *E. coli* PA14/17480-5-ovary (groups 1, 2 and 4; concentration: $4.2 \times 10^8$ CFU/ml) or with *E. coli* PA16/13200-animal2/clone3 (groups 3 and 5; concentration: $3.2 \times 10^8$ CFU/ml). The volumes used for inoculation or vaccination/challenge procedures were the same as described in trial 1. Following challenge, birds were sequentially killed at 3, 7, 10 and 14 dpc for necropsy and sampling. Clinical signs were observed daily. Blood samples were collected from surplus birds killed after hatch at 1 day of age, from all birds at the day of challenge and from necropsied birds at different sampling time points after challenge.

Table 2: Experimental design of animal trial 2

| group | vaccination[a] (1 day of age) | challenge (14 days of age)[b] | necropsy/sampling (days post challenge) | | | |
|---|---|---|---|---|---|---|
| | | | 3 | 7 | 10 | 14 |
| 1 (n=12) | irradiated *E. coli* (aerosol) | PA14/17480/5-ovary | 2[c] | 3 | 3 | 3 |
| 2 (n=12) | irradiated *E. coli* (oculonasal) | PA14/17480/5-ovary | 3 | 3 | 3 | 3 |
| 3 (n=12) | irradiated *E. coli* (aerosol) | PA16/13200-animal2/clone3 | 3 | 3 | 3 | 3 |
| 4 (n=12) | no | PA14/17480/5-ovary | 3 | 3 | 3 | 3 |
| 5 (n=12) | no | PA16/13200-animal2/clone3 | 3 | 3 | 3 | 3 |
| 6 (n=12) | no | no | 3 | 3 | 3 | 3 |

[a] irradiated *E. coli* PA14/17480/5-ovary was used for immunization at 1 day of age, [b] controls obtained PBS; [c] number of birds killed for sampling, one bird died before scheduled killing

**Histopathology**

Organ samples (lungs, airsac, spleen and heart) were collected during necropsy and fixed in 10% neutral buffered formalin. Tissues were embedded in paraffin and sectioned into 5 $\mu$m slices. Hema-toxylin and eosin staining was done following a routine protocol and a lesion scoring scheme was established to categorize the severity of microscopic lesions as 0, 1 and 2 (Table 3).

Table 3: Histopathology lesion scoring scheme

| score | organ | | | | |
|---|---|---|---|---|---|
| | lung | airsac | liver | spleen[a] | heart |
| 0 | normal | normal | normal | normal | normal |

(continued)

| score | organ | | | | |
| | lung | airsac | liver | spleen[a] | heart |
| --- | --- | --- | --- | --- | --- |
| 1 | infiltration of inflammatory cells into secondary bronchi or in lung tissue | focal to multifocal thickening with infiltration of inflammatory cells and /or edema | 1 - 2 foci of mononuclear cell infiltration | heterophilic infiltration (count, n = <15) | infiltration of inflammatory cells into epicardium |
| 2 | infiltration of inflammatory cells into secondary bronchi and lung tissue | diffuse thickness with severe infiltration of inflammatory cells | multifocal infiltration of inflammatory cells with hepatocellular necrosis | heterophilic infiltration (count n≥15) | infiltration of inflammatory cells into epicardium extending to myocardium |

[a] spleen samples were examined under 40x objective and an average of 8 images were considered for quantification of heterophils.

## Bacteriology

Lungs, liver and spleen were processed for CFU count while heart and air sac were subjected to direct plating. For bacterial re-isolation, organ samples were directly streaked on agar plates. Absolute bacterial quantification was determined by colony forming unit (CFU) count in duplicate plates at 10 fold dilutions. Following incubation of plates at 37°C for overnight, positive growth of *E. coli* was recoded (direct plating) or colonies were counted (CFU count). Mac-Conkey agar plates were used for the growth of *E. coli* at all sampling time points except in the animal trial 1 where the challenged strain *lux*-tagged *E. coli* PA14/17480-5-ovary was selectively grown on LB agar plates with erythromycin at 3, 7 and 10 dpc.

## Enzyme linked immunosorbent assay

Antibody titers in serum samples against two strains of *E. coli* used for challenge of birds were measured with separate in-house customized ELISAs applying a protocol as described before (Paudel et al., 2017). Briefly, 96-well ELISA plates were coated either with *E. coli* PA14/17480-5-ovary or with *E. coli* PA16/13200-animal2/clone3 depending on the type of serum tested. On the next day, plates were dried and each well was treated with blocking buffer for one hour. After removing the blocking buffer, test sera diluted at 1:200 were conjugated with a secondary antibody (Goat-Anti-Chicken IgG-HRP) at 1:5000 dilution. Color reaction was initiated by adding tetramethylbenzidine substrate and stopped with 0.5M $H_2SO_4$. ODs were measured with an ELISA reader at 450 nm.

## Statistical analysis

The pattern of *E. coli* positive or negative organs in birds in animal trial 2 was analyzed with logistic regression models comparing estimated marginal mean among different groups. Likewise, microscopic lesion scores were compared with cumulative link models after validating the criteria for the proportional odds assumption. The main objective was to evaluate whether different vaccination and challenge treatments lead to differences in lesion severity scores. Both these data analyses were performed in R program (R core team, 2020, R: A language and environment for statistical computing. R Foundation for Statistical Computing, Vienna, Austria. https://www.R-project.org). For interpretation, emean is 0 when the probability of positive and negative bacterial re-isolation or the observation of LS 1/2 or LS 0 are equal in a particular group. Negative emean denotes that likelihood of number of positive organs is less than negative re-isolation. Likewise, in histology scores, chance of having LS 1/2 is less than LS 0. Interpretation should be made exactly in opposite direction when emean shows positive values. The comparison of average OD values in alamarBlue assay and ELISA among groups were done using the t-test and one-way ANOVA with Tukey's post hoc test, respectively (IBM® SPSS® version 25; IBM cooperation, New York, USA).

**Results**

**In *vitro* characteristics of irradiated cells**

No growth of bacteria on agar plates even after enrichment was noticed, ensuring the complete loss of multiplication ability in irradiated cells.

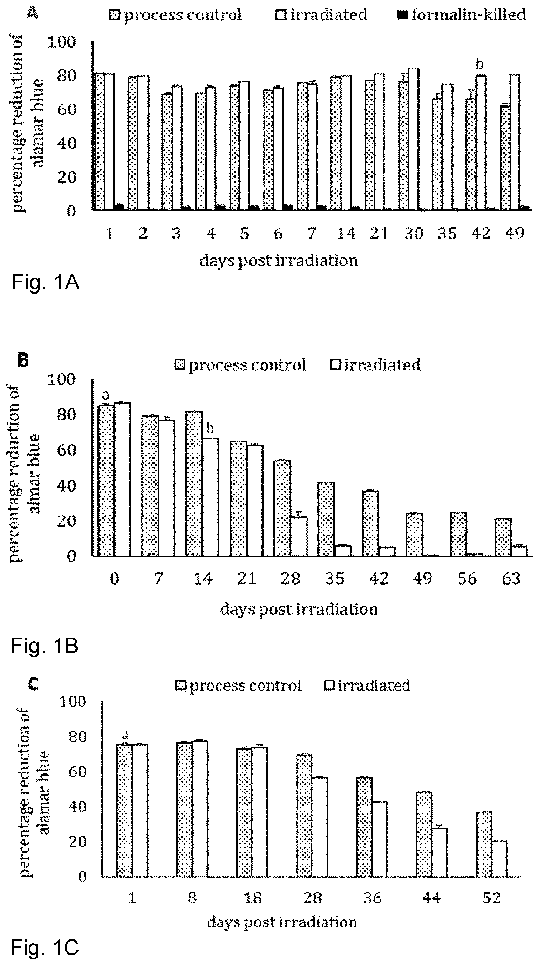

Long-term percentage reduction of alamarBlue in three separate experiments is shown in Figure 1. Results of three separate experiments showed that freshly prepared irradiated *E. coli* PA14/17480/5-ovary were metabolically active in the same way as non-irradiated process control cells and the pattern remained the same for at least two weeks when the suspensions were stored at 4°C. In contrary, almost no metabolic activity remained in formalin-killed cells (Figure 1A).

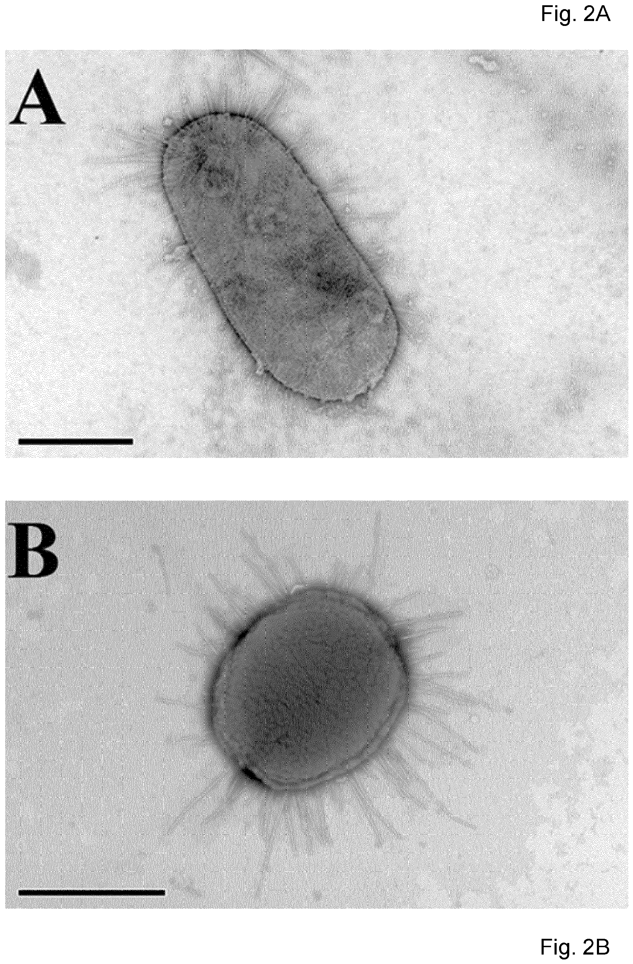

In the transmission electron microscopy, no differences were observed in bacterial cell integrity and fimbria of irradiated and non-irradiated process control cells, which demonstrated that outer cellular structure of *E. coli* is preserved after gamma irradiation. (Figure 2).

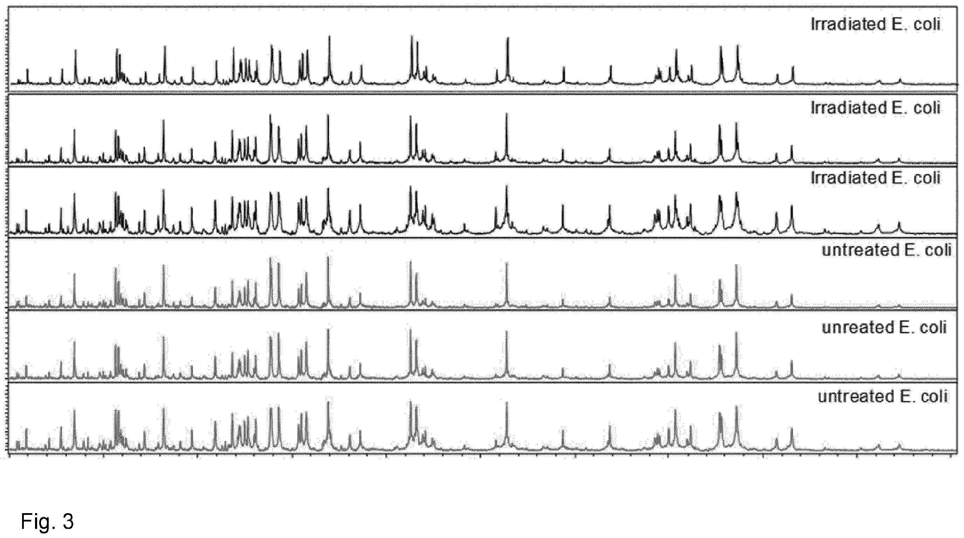

Upon analysis with MALDI-TOF MS, protein profile spectra of non-irradiated process control and irradiated PA14/ PA14/17480/5-ovary were very similar (Figure 3). All main peaks present in non-irradiated process control bacteria were also identified in irradiated ones. Consequently, both types of cells could produce same log score values for reliable species identification.

**In *vivo* experiments**

**Clinical signs**

In animal trial 1, mild drowsiness and ruffled feathers were observed in 2-5 birds/day/group in infected groups 2 and 3 staring from the first day until 16 days post infection (dpi). Such clinical signs were inconsistent and not always the same birds were affected. One bird from group 2 was euthanized at 13 dpi (23 days of age) due to the development of severe symptoms such as, difficulty in breathing, dropped wings and inability to move.

In animal trial 2, one bird from group 1 died at 5 days post vaccination. After challenge, 2-4 birds/day/group in groups 2, 4 and 5 showed inconsistent mild clinical signs as observed in trial 1. At 8 dpc, intense clinical signs started to progress in one bird from group 4 which was then killed at 10 dpc.

**Macroscopic lesions**

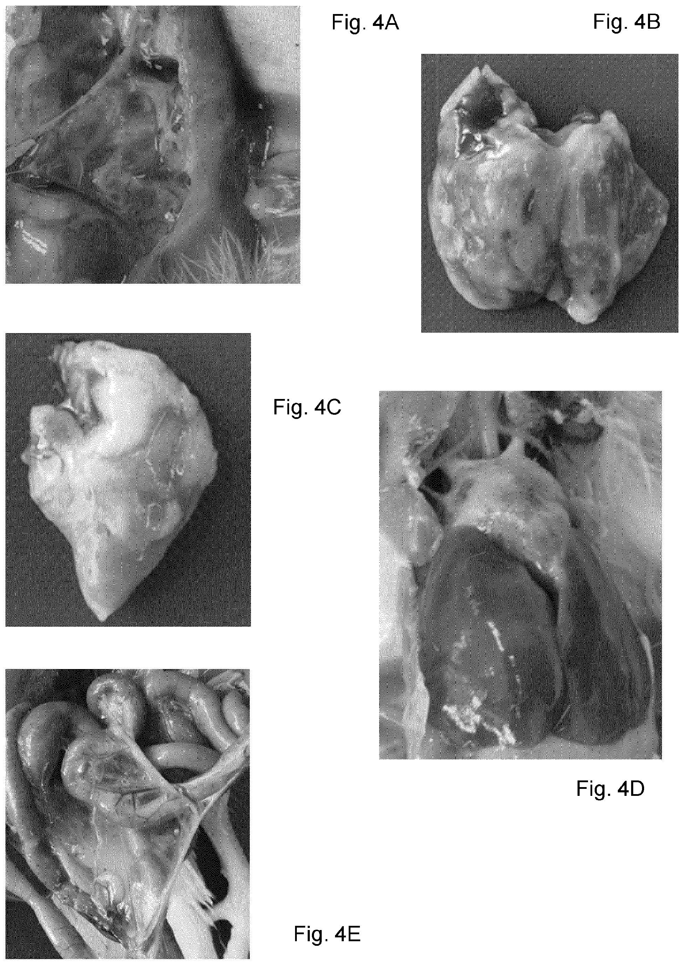

In animal trial 1, at 3 dpi, mild to severe colibacillosis-associated lesions such as, aerosaculitis, perihepatitis were seen in 4/4 and 3/4 birds from groups 2 and 3, respectively (Figure 4A). The bird that was euthanized at 13 dpi (23 days of age) had severe lesions in systemic organs, such as perihepatitis, pericarditis and aerosaculitis (Figure 4B, C). After challenge, 1 and 2 birds from group 2 showed mild pericarditis at 3 and 7 dpc respectively whereas fibrinous pericarditis (n=1) and cloudy airsac (n=2) were recorded in birds of group 5 at 7 dpc.

In animal trial 2, the bird which died at 5 dpc showed unabsorbed yolk sac. Following homologous challenge, no lesions were recorded in birds from group 1 expect in one with moderate aeroseculitis at 7 dpi. In group 2, mild to moderate aerosaculitis (n=4) and perihepatitis (n=1) were observed. In birds of group 3, no lesions were recorded. In the homologous challenge control group (group 4), lesions such as aerosaculitis (n=5), pericarditis (n=2), peritonitis (n=1) and perihepatitis (n=1) were observed (Figure 4D, E)=. Likewise, aerosaculitis (n=3), pericarditis (n=2) and/or perihepatitis (n=3) were also observed in the heterologous challenge control group (group 5) but severity of lesions was lower as compared to birds in group 4.

**Histopathology**

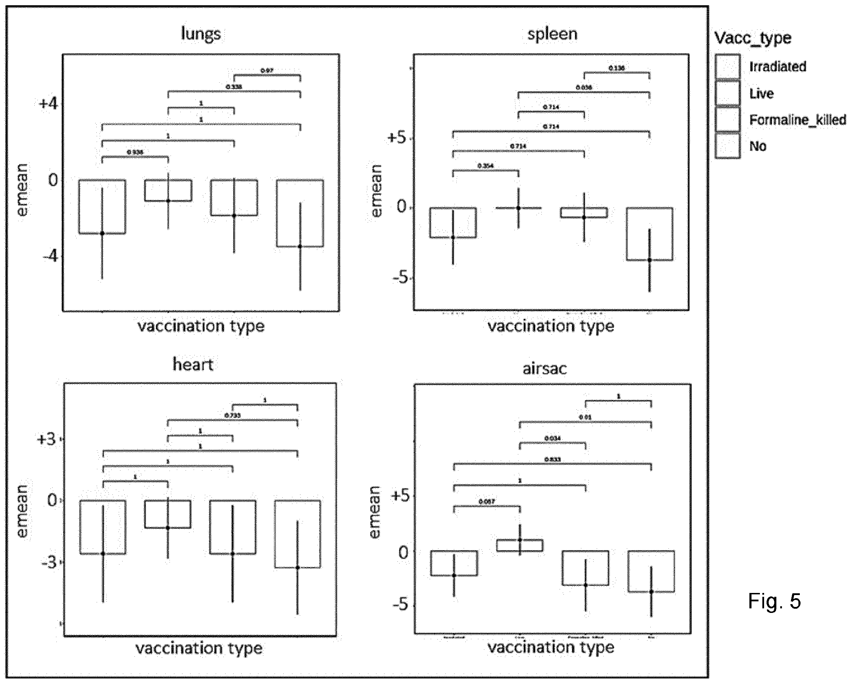

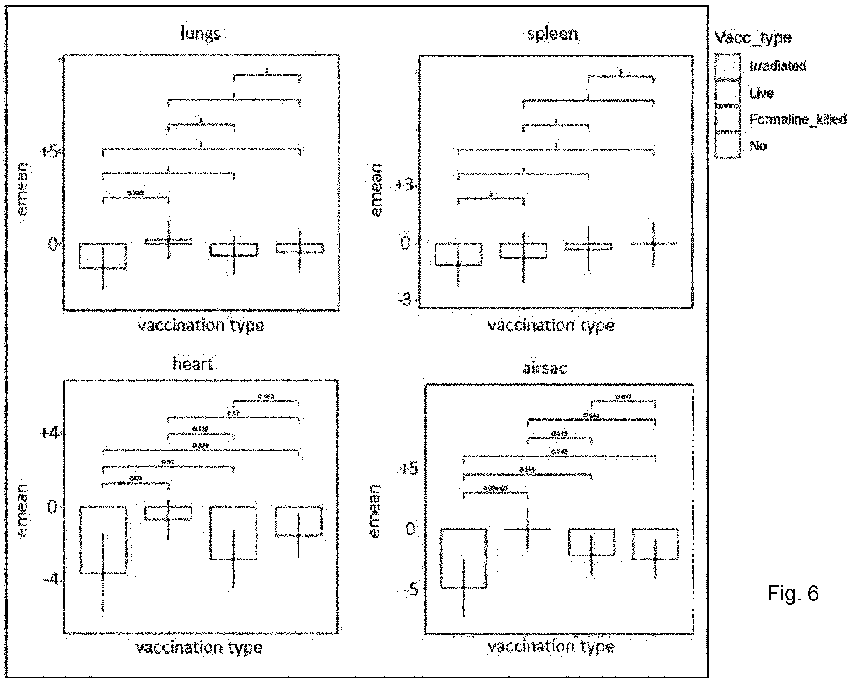

In animal trial 1, at 3 dpi, microscopic lesions were mostly seen in birds infected with live *E. coli* (Table 4). In group 1, all birds except one with LS 1 in air sac and spleen were devoid of pathological lesions. Average emean value in this group was very low in all organs compared to those infected with live *E. coli* (Figure 5), which proved the safety of the irradiated vaccine. After challenge, number of birds with LS 1 or 2 in systemic organs was the highest in group 5, which is the non-vaccinated challenge control (Table 4). The emean value was the lowest in group 1, which demonstrated a strong protection against challenge in birds that received the irradiated vaccine as compared to those that were immunized with live and formalin killed bacterial cells (Figure 6) .

Table 4: Histopathology lesion scores in animal trial 1

| groups | LS[a] | 3 days after first vaccination/infection | | | | after challenge | | | |
|---|---|---|---|---|---|---|---|---|---|
| | | lungs | spleen | heart | airsac | lungs | spleen | heart | airsac |
| 1 (irr. vaccinated + challenged) | 0 | 4[b] | 3 | 4 | 3 | 7 | 4 | 12 | 11 |
| | 1 | 0 | 1 | 0 | 1 | 4 | 8 | 0 | 1 |
| | 2 | 0 | 0 | 0 | 0 | 1 | 0 | 0 | 0 |
| 2 (infected + challenged)[c] | 0 | 4 | 2 | 5 | 0 | 2 | 4 | 5 | 1 |
| | 1 | 3 | 4 | 2 | 4 | 6 | 5 | 4 | 9 |
| | 2 | 1 | 2 | 1 | 4 | 3 | 2 | 2 | 1 |
| 3 (infected + not challenged) | 0 | NA[d] | NA | NA | NA | 7 | 4 | 8 | 4 |
| | 1 | | | | | 4 | 6 | 3 | 8 |
| | 2 | | | | | 1 | 2 | 1 | 0 |
| 4 (form. vaccinated + challenged) | 0 | 3 | 1 | 4 | 4 | 5 | 2 | 11 | 5 |
| | 1 | 1 | 3 | 0 | 0 | 5 | 9 | 1 | 7 |
| | 2 | 0 | 0 | 0 | 0 | 2 | 1 | 0 | 0 |
| 5 (challenged) | 0 | 8 | 8 | 8 | 8 | 5 | 2 | 8 | 6 |
| | 1 | 0 | 0 | 0 | 0 | 4 | 8 | 3 | 6 |
| | 2 | 0 | 0 | 0 | 0 | 3 | 2 | 1 | 0 |
| 6 (negative control) | 0 | NA | NA | NA | NA | 12 | 12 | 12 | 12 |
| | 1 | | | | | 0 | 0 | 0 | 0 |
| | 2 | | | | | 0 | 0 | 0 | 0 |

[a] for trial design and lesion scoring scheme refer to Tables 1 and 3, respectively; [b] number of birds with particular lesion score, scores of all birds killed after challenge were combined together; [c] one bird died at 13 days post infection and is not included in the total after challenge; [d] not applicable because before challenge, scores of birds of groups 3 and 6 were combined with groups 2 (infected) and 5 (non-infected), respectively for statistical analysis.

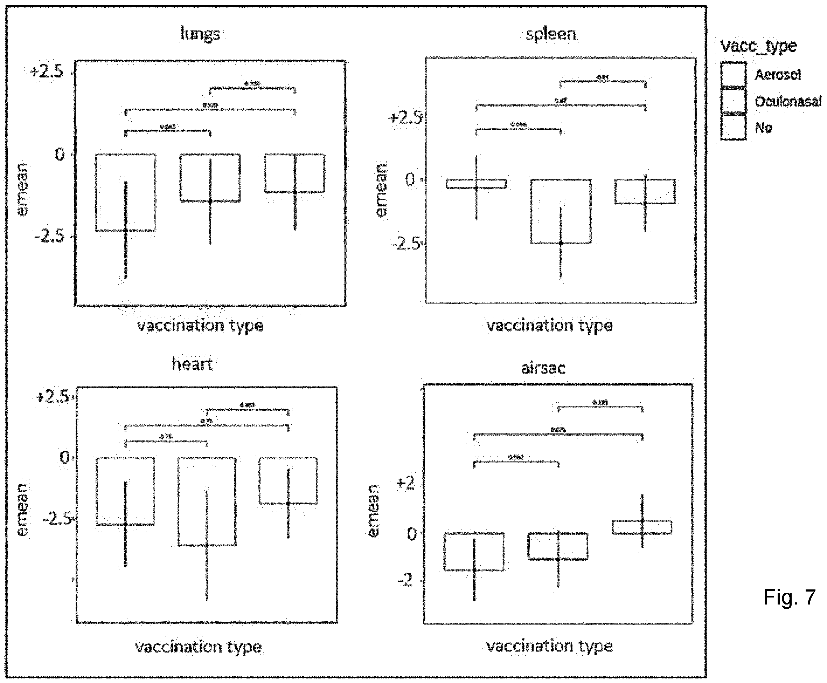

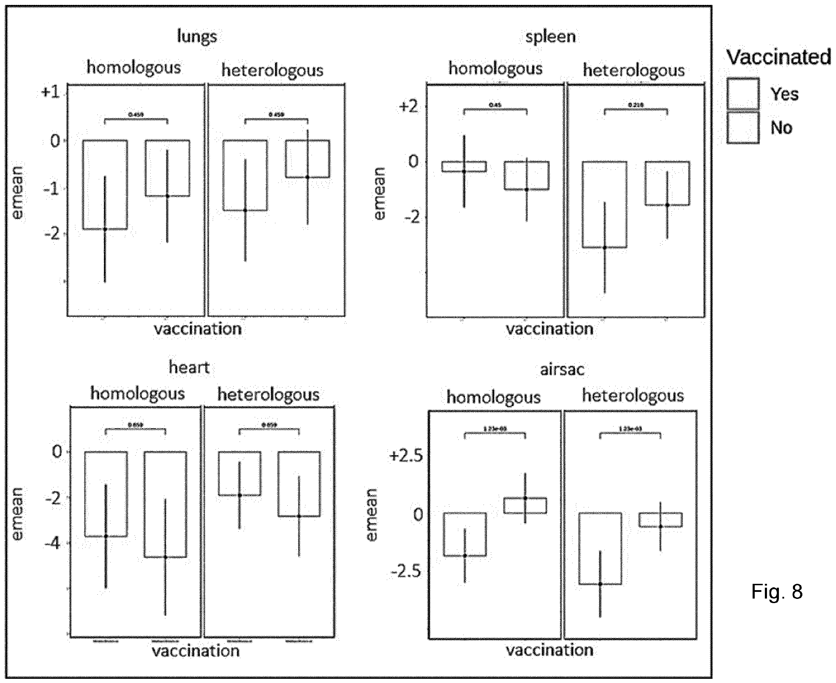

Histopathology lesion scores in birds in animal trial 2 are shown in Table 5. In comparison among groups 1 (vaccinated via aerosol route), 2 (vaccinated via oculonasal route) and 4 (homologous challenge control), emean values in lungs and airsac were the lowest in group 1 whereas in spleen the lowest value was observed in group 2 (Figure 7). Comparison of group 1 with group 4 (homologous challenge control) and group 3 with group 5 (heterologous challenge control) showed that lesions in lungs and airsac were reduced in vaccinated birds after challenge with both homologous and heterologous strains (Figure 8).

Table 5: Histopathology lesion scores in animal trial 2

| groups | LS[a] | after challenge | | | |
|---|---|---|---|---|---|
| | | lungs | spleen | heart | airsac |
| 1 (aerosol vaccinated + homologous challenged)[b] | 0 | 8[c] | 4 | 10 | 6 |
| | 1 | 3 | 4 | 1 | 4 |
| | 2 | 0 | 3 | 0 | 1 |
| 2 (oculonasal vaccinated + homologous challenged) | 0 | 7 | 9 | 12 | 5 |
| | 1 | 3 | 3 | 0 | 6 |
| | 2 | 2 | 0 | 0 | 1 |
| 3 (aerosol vaccinated + heterologous challenged) | 0 | 7 | 10 | 12 | 9 |
| | 1 | 3 | 2 | 0 | 3 |
| | 2 | 2 | 0 | 0 | 0 |

(continued)

| groups | LS[a] | after challenge | | | |
|---|---|---|---|---|---|
| | | lungs | spleen | heart | airsac |
| 4 (homologous challenged | 0 | 5 | 4 | 9 | 1 |
| | 1 | 7 | 8 | 2 | 8 |
| | 2 | 0 | 0 | 1 | 3 |
| 5 (heterologous challenged) | 0 | 6 | 6 | 10 | 2 |
| | 1 | 4 | 6 | 2 | 10 |
| | 2 | 2 | 0 | 0 | 0 |
| 6 (negative control) | 0 | 12 | 12 | 12 | 12 |
| | 1 | 0 | 0 | 0 | 0 |
| | 2 | 0 | 0 | 0 | 0 |

[a] for lesion scoring scheme refer to Table 2; [b] one bird died at 5 days post vaccination and is not included in the total after challenge; [c] number of birds with particular lesion score, all birds killed after challenge were combined together. For trial design refer to the Table 2.

**Bacteriology**

In animal trial 1, at 3 dpi (at 10 days of age), the highest frequency of *E. coli* positive organs were observed in birds from groups 2 and 3 (Table 6). In group 1, only one positive case was observed prior challenge in lungs while all other organs were negative. Pure colonies of *E. coli* were isolated from lungs, airsac, heart, and liver of the bird that was euthanized at 13 dpi. After challenge, the pattern of re-isolation was comparable in all vaccinated groups. Particularly in group 1 immunized with irradiated *E. coli,* 3, 2 and 1 birds were positive for *lux*-tagged PA14/17480/5-ovary in lungs, airsac and blood, respectively while all other systemic organs were negative. In contrast, 5, 1, 1, 3, 4 and 5 birds were positive in lungs, airsac, heart, liver, spleen and blood of birds in group 5, which showed that frequency of lungs positive and the pattern of systemic spread was higher in challenge controls than in vaccinated birds.

Table 6: Number of E. coli positive organs in animal trial 1

| groups | 3 days after first vaccination/ infection | | | | | after challenge | | | | | |
|---|---|---|---|---|---|---|---|---|---|---|---|
| | lungs | airsac | heart | liver | spleen | lungs | airsac | heart | liver | spleen | blood |
| 1 | 1/4[a] | 0/4 | 0/4 | 0/4 | 0/4 | 3/12[b] | 2/12 | 0/12 | 0/12 | 0/12 | 1/12 |
| 2[c] | 4/4 | 4/4 | 3/4 | 2/4 | 4/4 | 3/11[c] | 0/11 | 0/11 | 0/11 | 0/11 | 1/11 |
| 3 | 2/4 | 4/4 | 3/4 | 2/4 | 4/4 | NA | NA[d] | NA | NA | NA | NA |
| 4 | 2/4 | 1/4 | 0/4 | 1/4 | 1/4 | 2/12 | 1/12 | 0/12 | 0/12 | 0/12 | 3/12 |
| 5 | 1/4 | 1/4 | 0/4 | 0/4 | 0/4 | 5/12 | 1/12 | 1/12 | 3/12 | 4/12 | 5/12 |
| 6 | 2/4 | 0/4 | 0/4 | 0/4 | 0/4 | 3/12 | 2/12 | 0/12 | 0/12 | 0/12 | 1/12 |

[a] number of *E. coli* positive birds in respective organs/number of birds sampled; [b] number of birds positive for lux-tagged PA14/17480/5-ovary in respective organs/number of birds samples after challenge; [c] one bird was euthanized before scheduled killing due to the onset of severe clinical signs; [d] not applicable. For trial design refer to the Table 1.

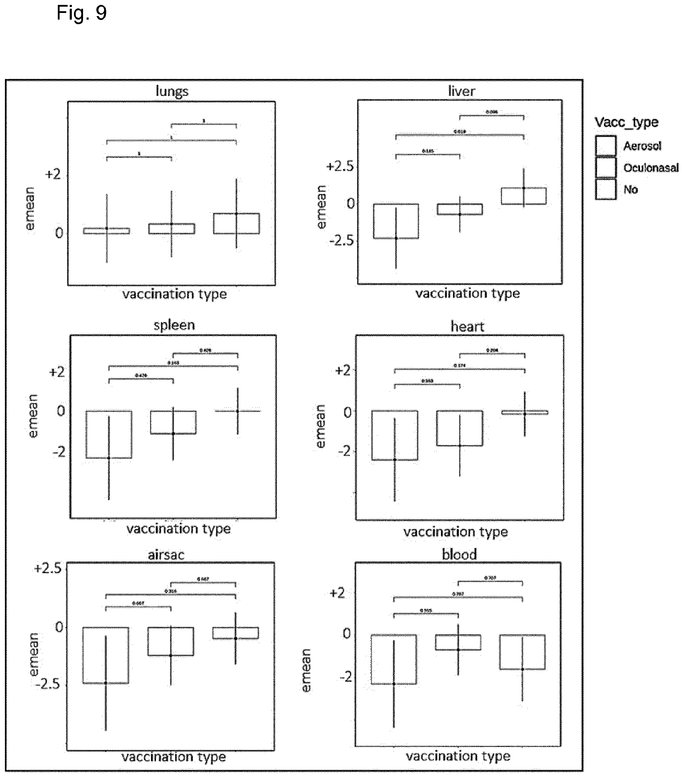

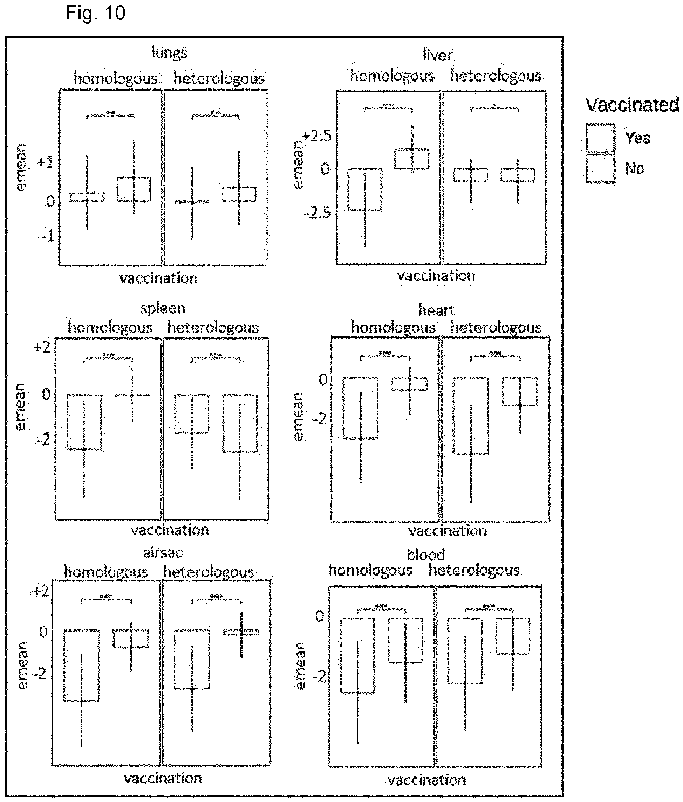

In animal trial 2, *Pseudomonas aeruginosa* was cultured from yolk sac of the bird from group 1 that died at 5 days post vaccination. After challenge, lungs were mostly positive in all challenged birds, irrespective of their vaccination status (Table 7). All but one bird vaccinated via aerosol route were negative in bacterial re-isolation in systemic organs. The finding from the logistic pattern analysis is shown in Figures 9 and 10. The likelihood of bacterial re-isolation was the highest in group 4 (Figure 9). It also showed that aerosol vaccination is the preferred route of vaccine delivery as compared to oculonasal route. Furthermore, comparison of vaccinated groups with respective challenge controls (group 1 with 4 and group 3 with 5) showed that the likelihood of positive re-isolation from lungs, liver, heart, airsac and blood was less in vaccinated groups than in challenge control (Figure 10) .

**Table 7: Number of E. coli positive organs in animal trial 2**

| groups | | | after challenge | | | |
|---|---|---|---|---|---|---|
| | lungs | airsac | heart | liver | spleen | blood |
| 1 | 6/11[a] | 0/11 | 0/11 | 1/11 | 1/11 | 1/11 |
| 2 | 7/12 | 2/12 | 1/12 | 4/12 | 3/12 | 4/12 |
| 3 | 6/12 | 1/12 | 1/12 | 4/12 | 2/12 | 1/12 |
| 4 | 8/12 | 4/12 | 5/12 | 9/12 | 6/12 | 2/12 |
| 5 | 7/12 | 5/12 | 2/12 | 4/12 | 1/12 | 3/12 |
| 6 | 1/12 | 0/12 | 0/12 | 0/12 | 0/12 | 0/12 |

[a] number of *E. coli* positive birds in respective organs/number of birds sampled, one bird from group 1 died before scheduled killing. For trial design refer to the Table 2.

In trial 2, in aerosol vaccinated and challenged birds from groups 1 and 3, all except one bird were devoid of macroscopic lesions, in contrast to their respective challenge controls. Furthermore, likelihood of microscopic lesion score 0 in lungs and airsac as well as negative re-isolation in organs and blood were higher. Thus, the protection of vaccinated birds against homologous and heterologous challenge was demonstrated.

**Antibody titers**

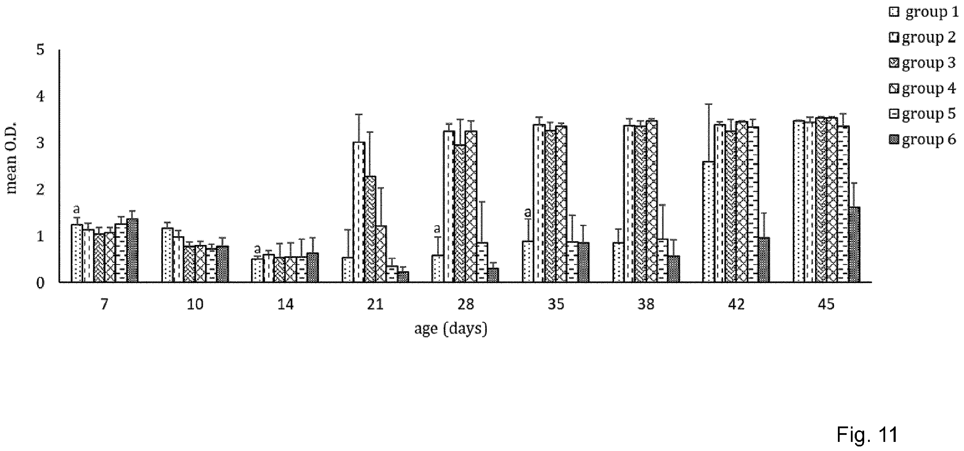

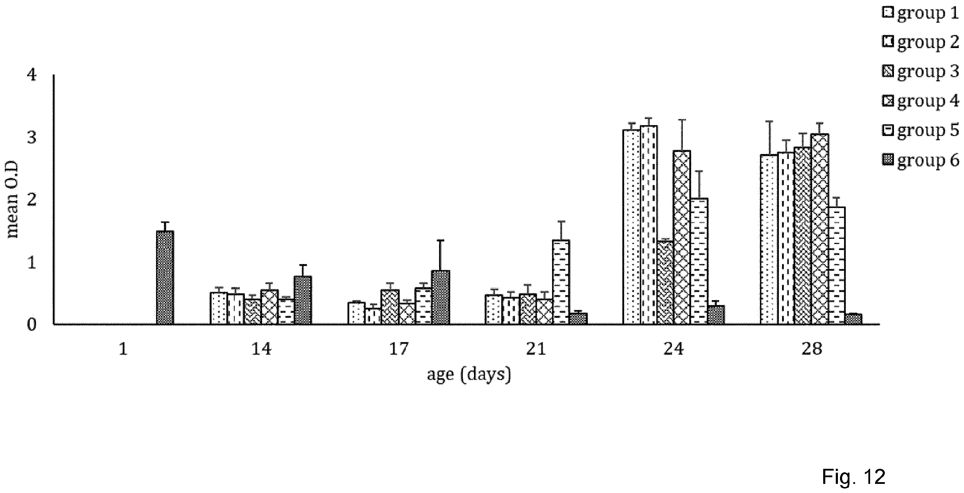

Antibody titers against *E. coli* challenge strains measured in animal trials 1 and 2 are shown in Figures 11 and 12, respectively. In animal trial 1, unlike in groups 2, 3, and 4, antibody titers were not influenced due to immunization of birds with irradiated *E. coli* in birds of group 1 (Figure 11). After one week post challenge, the antibody titer reached to the maximum in all challenged birds. In birds of group 2, which were infected with live *E. coli* at 7 days, the maximum antibody titers were maintained until the end of experiment.

In trial 2, similar results were obtained as in trial 1. Antibody titers were not provoked due to vaccination but increased after challenges were made. When coating was done with PA14/17480/5-ovary and sera of birds challenged with PA16/13200-animal2/clone 3 tested, no cross reactivity was noticed. Thus, coating with homologous strain was done to test the sera of different groups depending on the type of strains used for challenge.

**Selection of a new heterologous challenge strain (animal trial 3):**

Three O78:K80 isolates that caused the highest mortality rates in embryos were selected to perform an animal trial and to elucidate pathogenicity *in vivo.* The bacterial culture was washed and resuspended in phosphate buffered saline (PBS) before infection.

In total, 60 SPF layer chickens were hatched and used in the trial. At the age of 14 days, birds in groups 1-4 were infected with different *E. coli* strains via aerosol route as shown in Table 8. The procedure of aerosol infection is also the same as we did in our previous experimental infection study with *E. coli.* At this time, sterile PBS was administered in control birds of group 5. Four birds were euthanized at weekly intervals post infection for necropsy and sampling.

**Table 8: Trial design**

| Group | *E. coli* strain used for infection |
|---|---|
| 1 | PA14/17480/5-ovary (O1:K1) |
| 2 | PA15/19103-3 (078:K80) |
| 3 | PA15/25396-3 right (O78:K80) |
| 4 | PA15/24960-2 (O78:K80) |
| 5 | no |

**Table 9: Clinical score**

| group | Clinical score | | |
|---|---|---|---|
| | Score 1 | Score 2 | Score 3 |
| Week 1 post cha llenge | | | |
| 1 | 4/12 | 1/12 | 0/12 |
| 2 | 5/12 | 1/12 | 0/12 |
| 3 | 2/12 | 0/12 | 0/12 |
| 4 | 3/12 | 1/12 | 0/12 |
| 5 | 0/12 | 0/12 | 0/12 |
| Week 2 post cha llenge | | | |
| 1 | 0/8 | 1/8 | 0/8 |
| 2 | 3/8 | 0/8 | 0/8 |
| 3 | 1/8 | 0/8 | 0/8 |
| 4 | 2/8 | 0/8 | 0/8 |
| 5 | 0/8 | 0/8 | 0/8 |
| Week 3 post cha llenge | | | |
| 1 | 0/4 | 0/4 | 0/4 |
| 2 | 0/4 | 0/4 | 0/4 |
| 3 | 1/4 | 0/4 | 0/4 |
| 4 | 0/4 | 0/4 | 0/4 |
| 5 | 0/4 | 0/4 | 0/4 |

**Table 10: Macroscopic lesions**

| group | lesions | | | |
|---|---|---|---|---|
| | lung | Air sac | heart | liver |
| 1 | ≤ 1/2[a] of the of the lung (7) ≥ 1/2 of the of the lung (2) | Slight opaque (5) Severe airsaculitis (1) | Opacity with lack of transparency (3) Marked pericarditis (1) | Slight amount of fi-brin (4) Marked perihepatitis (1) |
| 2 | ≤ 1/2 of the of the lung (7) ≥ 1/2 of the of the lung (2) | Slight opaque (4) Moderate opaque (2) Severe airsaculitis (2) | Opacity with lack of transparency (5) Marked pericarditis (3) | Slight amount of fibrin (2) Marked perihepatitis (1) |
| 3 | ≤ 1/2 of the of the lung (7) ≥ 1/2 of the of the lung (2) | Slight opaque (6) | Opacity with lack of transparency (6) | Slight amount of fibrin (3) |
| 4 | ≤ 1/2 of the of the lung (5) ≥ 1/2 of the of the lung (3) | Slight opaque (7) severe airsaculitis (1) | Opacity with lack of transparency (5) Marked pericarditis (2) | Slight amount of fibrin (4) Marked perihepatitis (1) |
| 5 | - | - | - | - |

**Table 11: Direct plating for E. coli isolates**

| Group | lung | air sac | heart | liver | spleen |
|---|---|---|---|---|---|
| 1 | 3 (615, 616, 619) | 2 (619, 621) | 1 (622) | 1 (622) | 1 (622) |
| 2 | 3 (625, 626, 627) | 4 (625, 626, 627, 635) | 2 (625, 627) | 2 (625, 634) | 3 (627, 634, 635) |
| 3 | 2 (640, 644) | 4 (638, 639, 640, 644) | 1 (640) | 1 (644) | 1 (640) |
| 4 | 1 (650) | 1 (650) | 2 (650, 658) | 1 (650) | 1 (650) |
| 5 | | | | | |

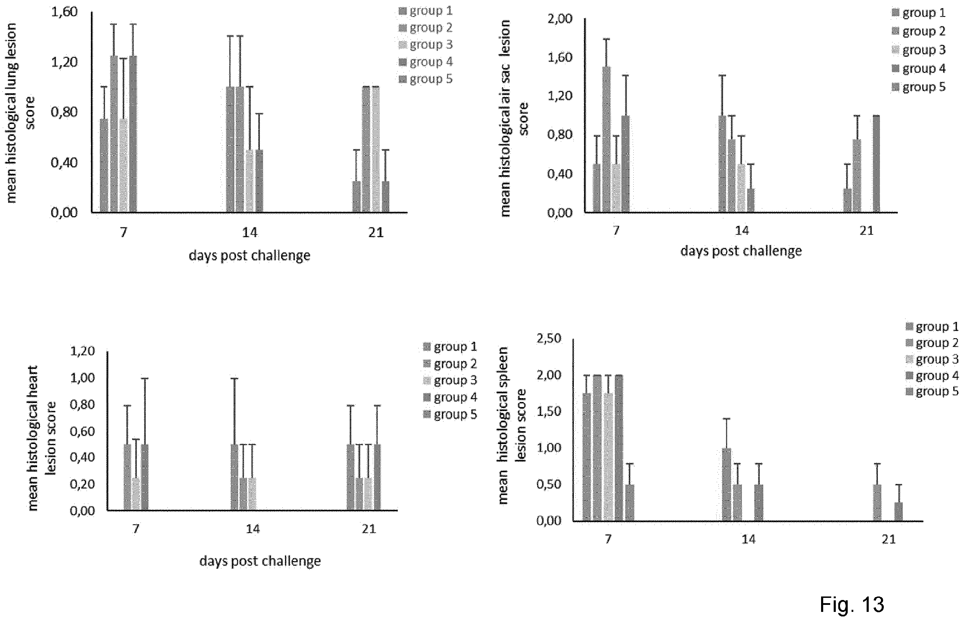

The histopathology is depicted in Fig. 13: Group 1: PA14/17480/5-ovary; group 2: PA 15/19103-3, group 3: 15/25396-3 right, group 4: 15/24960-2, group 5: negative control

**Conclusion from Trial:**

The isolate *E. coli* 15/19103-3 (O78:K80) has been identified as being suitable for heterologous challenge based on clinical signs (more number of birds are affected), macroscopic lesions (more birds showed severe lesions), bacteriology (reisolation is much higher in this group) histopathology (lesions in lungs and airsac are higher).

**Animal trial on heterologous challenge (animal trial 4):**

The next animal trial was conducted to evaluate the efficacy of vaccination with irradiated *E. coli* cells against O78:K80 strain, namely isolate PA15/19103-3 from the previous trial. It was already demonstrated that birds were significantly protected against homologous challenge (O1:K1) (see above). The findings from the actual study should evaluate the broader protection ability of irradiated *E. coli* cells against a pathogenic isolate belonging to O78:K80 serotype. Furthermore, it should show the capability of strain PA15/19103-3 to protect against a homologous challenge, which was not very successful.

**Table 12: Experimental design**

| groups | number of birds | vaccination[a] | challenge[b] | killing[c] |
|---|---|---|---|---|
| 1 | 16 | Irradiated PA14/17480/5ovary (O1:K1) | PA14/17480/5ovary (O1:K1) | 4 birds on the day of challenge and 4 birds atat 5, 8 and 14 dpc |
| 2 | 12 | Irradiated PA14/17480/5ovary (O1:K1) | *E. coli* PA15/19103-3 (O78:K80) | 4 birds on the day of challenge and 4 birds at 5, 8 and 14 dpc |
| 3 | 16 | Irradiated PA15/19103-3 (O78:K80) | *E. coli* PA15/19103-3 (O78:K80) | 4 birds on the day of challenge and 4 birds atat 5, 8 and 14 dpc |
| 4 | 12 | no | PA14/17480/5ovary (O1:K1) | 4 birds at 5, 8 and 14 dpc |
| 5 | 12 | no | *E. coli* PA15/19103-3 (O78:K80) | 4 birds at 5, 8 and 14 dpc |
| 6 | 16 | no | no | 4 birds on the day of challenge and 4 birds atat 5, 8 and 14 dpc |

[a] vaccination at the first day of life; [b] challenge at 14 days of life; [c] dpc: days post challenge

**Results:**

**alamarBlue Assay:**

The percentage reduction of process control (live) and irradiated PA15/19103-3 as determined with alamarBlue assay was much lower, which was very contrasting to PA14/17480/5-ovary.

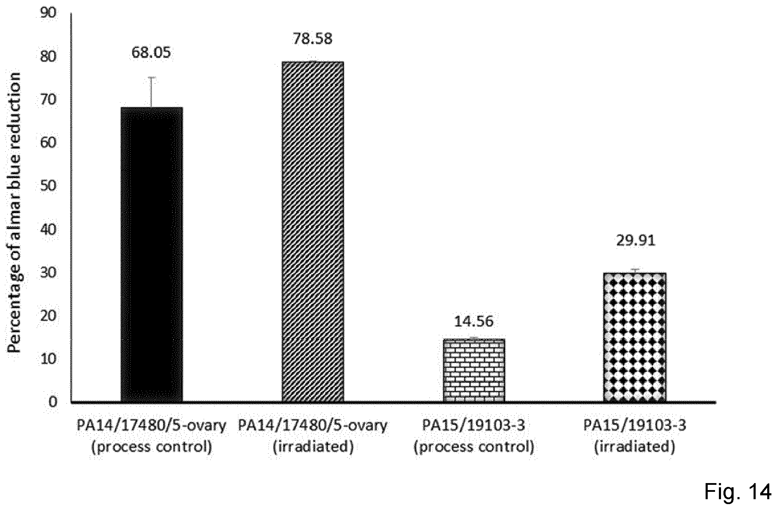

The results are depicted in Figure 14 ("Determination of percentage reduction of alamarBlue in two *E. coli* isolates of different serotypes) and show the following values: PA14/17480/5-ovary (process control): 68.05 %, PA14/17480/5-ovary (irradiated): 78.58, PA15/19103-3 (process control): 14.56, and PA15/19103-3 (irradiated): 29.91.

**Table 13: Clinical signs:**

| Group[a] | Clinical score | | | |
|---|---|---|---|---|
| | 0 | 1 | 2 | 3 |
| 1 (n= 12) | 12[b] | 0 | 0 | 0 |
| 2 (n= 12) | 11 | 1 | 0 | 0 |
| 3 (n= 12) | 9 | 2 | 1 | 0 |
| 4 (n= 12) | 7 | 4 | 1 | 0 |
| 5 (n= 12) | 8 | 3 | 1 | 0 |

(continued)

| Group[a] | Clinical score | | | |
|---|---|---|---|---|
| | 0 | 1 | 2 | 3 |
| 6 (n= 12) | 12 | 0 | 0 | 0 |

[a] Group 1: vaccinated with O1:K1 and challenged with O1:K1; group 2: vaccinated with O1:K1 and challenged with O78:K80; group 3: vaccinated with O78:K80 and challenged with O78:K80; group 4: positive (challenge) control O1:K1; group 5: challenge (positive) control O78:K80; group 6: negative control. [b] Number of birds/ score

The lowest number of chickens with clinical signs were noticed in homologous challenged group 1 (O1:K1), similar to the negative control group 6. Next to this, group 2 with heterologous challenge showed only a single bird with weak clinical signs (score 1). In comparison, most severe clinical signs were noticed in group 4, O1:K1 challenge control.

**Table 14: Macroscopic lesions:**

| dpi[a] | Group[b] | Airsacculitis | | | Pericarditis | | | Perihepatitis | | | peri-toni-tis | Lung lesion |
|---|---|---|---|---|---|---|---|---|---|---|---|---|
| | | mild | moderate | severe | mild | moderate | severe | mild | moderate | severe | | |
| 5 | 1 | - | - | - | - | - | - | - | - | - | - | - |
| | 2 | - | 2[c] | -[d] | - | - | - | 1 | - | - | - | - |
| | 3 | - | - | - | - | - | - | - | - | - | - | - |
| | 4 | 1 | 2 | - | - | - | - | 2 | - | - | 2 | - |
| | 5 | 2 | 1 | - | 1 | - | - | 2 | 1 | - | - | - |
| | | - | - | - | - | - | - | - | - | - | - | - |
| 8 | 1 | - | - | - | - | - | - | - | - | - | - | - |
| | 2 | - | 1 | 1 | - | 1 | 1 | - | - | 1 | - | - |
| | 3 | 1 | 1 | 1 | 1 | 1 | 1 | - | 1 | - | - | - |
| | 4 | 1 | - | - | - | 1 | - | - | - | 1 | - | - |
| | 5 | 3 | - | - | - | 1 | - | - | - | - | - | 2 |
| | 6 | - | - | - | - | - | - | - | - | - | - | - |
| 14 | 1 | - | - | - | - | - | - | - | - | - | - | - |
| | 2 | - | - | - | - | - | - | - | - | - | - | - |
| | 3 | 1 | - | - | - | 1 | - | - | - | 1 | - | - |
| | 4 | - | - | - | - | - | - | - | - | - | - | 1 |
| | 5 | 4 | - | - | - | 1 | - | - | - | - | - | 2 |
| | 6 | - | - | - | - | - | - | - | - | - | - | - |

[a] dpi: days post infection. [b] Group 1: vaccinated with O1:K1 and challenged with O1:K1; group 2: vaccinated with O1:K1 and challenged with O78:K80; group 3: vaccinated with O78:K80 and challenged with O78:K80; group 4: positive control O1:K1 challenge (positive) control; group 5: challenge (positive) control O78:K80; group6: negative control; [c] Number of birds/ lesion. [d] No lesion observed.

At 5dpi no lesions were noticed in homologous (O1:K1) challenged birds (group 1) whereas 2 birds showed moderate airsaculitis and 1 bird a mild perihepatitis in heterologous challenged birds (group 2). Birds in groups 4 and 5 showed most severe lesions, which was noticed until the end of the trial. At 8 dpi no lesions were noticed in birds from group1, similar to the negative control. At 14dpi no lesions were noticed in birds vaccinated with O1:K1 independent of the challenge (homologous or heterologous). Most severe lesions were noticed in non-vaccinated challenge controls but some minor lesions were also noticed in birds vaccinated with O78:K80 and challenged homologous.

**Table 15: Summary of lesion scores (independent of day post challenge)**

| Group[a] | Macroscopic lesions | | | | | | | | | | |
|---|---|---|---|---|---|---|---|---|---|---|---|
| | Airsacculitis | | | Pericarditis | | | Perihepatitis | | | peri- | |
| | mild | moderate | severe | mild | moderate | severe | mild | moderate | severe | toni-tis | Lung lesion |
| 1 | - | - | - | - | - | - | - | - | - | - | - |
| 2 | - | 3[b] | 1[c] | - | 1 | 1 | 1 | - | 1 | - | - |
| 3 | 2 | 2 | 1 | 2 | 1 | 1 | 1 | - | - | - | - |
| 4 | 2 | 2 | 1 | - | - | 1 | 3 | - | 1 | 3 | 1 |
| 5 | 9 | 1 | | 3 | - | - | 2 | 1 | - | - | 4 |
| 6 | - | - | - | - | - | - | - | - | - | - | - |

[a] Group 1: vaccinated with O1:K1 and challenged with O1:K1; group 2: vaccinated with O1:K1 and challenged with O78:K80; group 3: vaccinated with O78:K80 and challenged with O78:K80; group 4: positive control O1:K1 challenge (positive) control; group 5: challenge (positive) control O78:K80; group6: negative control; [c] Number of birds/ lesion. [d] No lesion observed.

Most lesions were noticed in challenge controls groups 4 and 5. Lesions in the lung were not seen in any of the birds vaccinated with O1:K1 (groups 1 and 2) or O78:K80 (group 3). Surprisingly, birds vaccinated with O78:K80 and homologously challenged were not completely protected similar to birds vaccinated with O1:K1 and challenged with O78:K80. However, most severe lesions were noticed in birds from group 5 which were substantially reduced by heterologous vaccination.

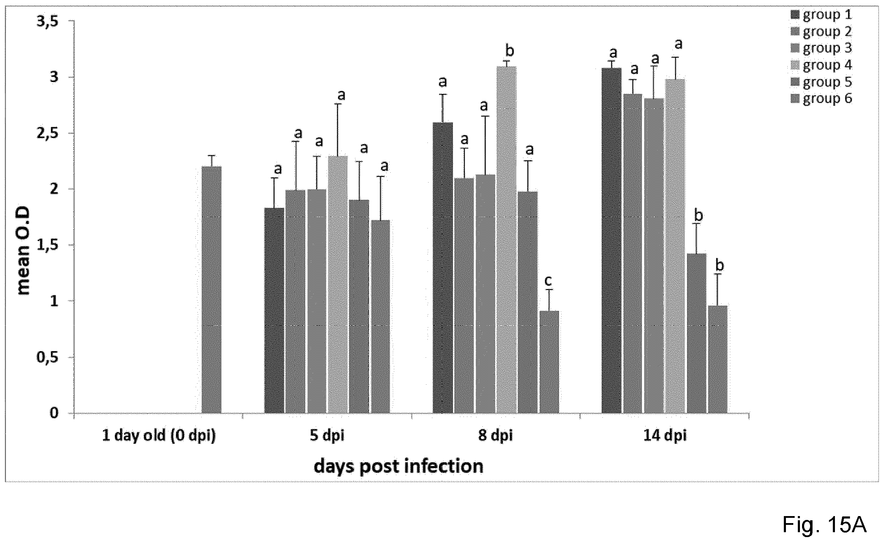

The results for the serology are depicted in Fig. 15: Fig. 15A shows the antigen PA14/17480/5-ovary (O1:K1). Group 1: vaccinated with O1:K1 and challenged with O1:K1; group 2: vaccinated with O1:K1 and challenged with O78:K80; group 3: vaccinated with O78:K80 and challenged with O78:K80; group 4: positive control O1:K1 challenge (positive) control; group 5: challenge (positive) control O78:K80; group6: negative control; groups with different superscripts are statistically different; Fig. 15 B shows the antigen: 15/19103-3 (O78:K80). Group 1: vaccinated with O1:K1 and challenged with O1:K1; group 2: vaccinated with O1:K1 and challenged with O78:K80; group 3: vaccinated with O78:K80 and challenged with O78:K80; group 4: positive control O1:K1 challenge (positive) control; group 5: challenge (positive) control O78:K80; group6: negative control; groups with different superscripts are statistically different

### Summary of Serology:

Homologous antigen with higher OD values; O78:K80 more specific; b) strong increase already 5dpi in group 5 with heterologous challenge control compared to vaccinated - protected - birds. Slightly lower antibodies in group 2 compared to group 3 at 5dpi indicate a better protection. At 8dpi, no difference anymore and even group 4 (challenged with O1:K1) with high levels at 14dpi.

### Bacteriology:

Pattern of bacterial isolation in different organs of birds from groups 2 (vaccinated with irradiated PA14/17480/5-ovary), 3 (vaccinated with irradiated PA15/19103-3) and 5 (not vaccinated) is shown in Figure 16. All the groups were challenged with PA15/19103-3. Results showed that mean values in group of birds vaccinated with irradiated PA15/19103-3 and challenged with the same strain was higher as compared to those that were vaccinated with PA14/17480/5-ovary and or challenged only with PA15/19103-3.

It demonstrated the lack of protection efficacy of the irradiated suspension prepared from the strain PA15/19103-3 even against the homologous challenge.

Fig. 16 shows the pattern of bacterial re-isolation from different organs of birds that were vaccinated with irradiated PA14/17480/5-ovary (O1:K1) or irradiated PA15/19103-3 (O78:K80) or left without vaccination. All birds were challenged with PA15/19103-3.

Pattern of bacterial reisolation in birds of groups 1, 2, 4 and 5 are shown in Fig. 17. The first two groups were vaccinated with PA14/17480/5-ovary but challenged with two different E. coli strains belonging to different serotypes. The mean values in all the organs in these two groups were much lower than their respective challenge controls (groups 4 and 5) which showed that colonization of challenge strains in birds vaccinated with irradiated PA14/17480/5-ovary is very limited.

Fig. 17 shows the pattern of bacterial re-isolation in birds of groups 1, 2, 4 and 5. Groups 1 and 2 were vaccinated with irradiated PA14/17480/5-ovary and challenged with PA14/17480/5-ovary (O1:K1) or PA15/19103-3 (O78:K80), respectively. Groups 4 and 5 were left without vaccination and challenged PA14/17480/5-ovary or PA15/19103-3, respectively.

Histopathology:

Pattern of occurrence of histopathological lesions in groups 2, 3 and 5, that were vaccinated with PA14/17480/5-ovary (O1:K1), PA15/19103-3 (O78:K80) or inoculated with PBS is shown in Fig. 18. The challenge strain, PA15/19103-3 (O78:K80) remained constant in all these groups. Results showed that pattern of histopathological lesions in group 2 was the least in lungs, spleen, heart and airsac followed by groups 3 and 5. This indicated again that vaccination with PA15/19103-3 and challenged with homologous strain worked less well that heterologous protection in group 2.

A similar comparison among groups 1 and 4 as well as 2 and 5 are shown in Fig. 19. Here, the vaccination strain (PA14/17480/5-ovary is the same in both vaccinated groups 1 and 2 but different challenge strains; groups 4 and 5 were challenged with homologous and heterologous strains (PA15/19103-3), respectively, as challenge controls. Histology scores, most prominently in lungs and airsacs were lower in vaccinated birds after homo-or heterologous challenge.

Fig. 18 shows the pattern of histology lesion scores in different organs of birds that were vaccinated with irradiated PA14/17480/5-ovary (O1:K1) or irradiated PA15/19103-3 (O78:K80) or left without vaccination. All birds were challenged with PA15/19103-3.

Fig. 19 shows the pattern of histology lesion scores in birds of groups 1, 2, 4 and 5. Groups 1 and 2 were vaccinated with irradiated PA14/17480/5-ovary and challenged with PA14/17480/5-ovary (O1:K1) or PA15/19103-3 (O78:K80), respectively. Groups 4 and 5 were left without vaccination and challenged PA14/17480/5-ovary or PA15/19103-3, respectively.

**Overall summary of heterologous trial:**

- Reconfirmation that strain PA14/17480/5-ovary protected well against homologous challenge
- Vaccination with PA15/19103-3 and challenged with homologous strain worked less well than heterologous protection in birds vaccinated with strain PA14/17480/5-ovary.
- Higher metabolic activity (alamarBlue) of strain PA14/17480/5-ovary (O1:K1) than PA15/19103-3 (O78:K80): explanation for better protection? Consequently, not each strain can be used and will deliver results as PA14/17480/5-ovary.

**References**

Abd El-Mawgoud AI, El-Nahass ES, Shany SAS, et al. (2020) Efficacy of live attenuated vaccine and commercially available lectin against avian pathogenic E. coli infection in broiler chickens. Veterinary Sciences. DOI: 10.3390/VETSCI7020065.

Alber A, Stevens MP and Vervelde L (2021) The bird's immune response to avian pathogenic Escherichia coli. Avian Pathology. DOI: 10.1080/03079457.2021.1873246.

Corbanie EA, Matthijs MGR, Van Eck JHH, et al. (2006) Deposition of differently sized airborne microspheres in the respiratory tract of chickens. Avian Pathology. DOI: 10.1080/03079450601028845.

Fertey et el., Viruses 8 (2016), 319.

Filho TF, Fávaro C, Ingberman M, et al. (2013) Effect of spray escherichia coli vaccine on the immunity of poultry. Avian Diseases. DOI: 10.1637/10456-112612-ResNote.1.

Gharib A, Hamouda A, bdel-Wahab AA, et al. (2017) Protective Efficacy of a Commercial Live Attenuated aroA mutant Vaccine Against Avian Pathogenic Escherichia coli Challenge in Broilers. Zagazig Veterinary Journal. DOI: 10.21608/zvjz.2017.7867.

Ghunaim H, Abu-Madi MA and Kariyawasam S (2014) Advances in vaccination against avian pathogenic Escherichia coli respiratory disease: Potentials and limitations. Veterinary Microbiology. DOI: 10.1016/j.vetmic.2014.04.019.

Gregersen RH, Christensen H, Ewers C, et al. (2010) Impact of Escherichia coli vaccine on parent stock mortality, first week mortality of broilers and population diversity of E. coli in vaccinated flocks. Avian Pathology. DOI: 10.1080/03079457.2010.495744.

Hellfritzsch M and ScherlieB R (2019) Mucosal vaccination via the respiratory tract. Pharmaceutics. DOI:

10.3390/pharmaceutics11080375.

Johnson TJ, Kariyawasam S, Wannemuehler Y, et al. (2007) The genome sequence of avian pathogenic Escherichia coli strain O1:K1:H7 shares strong similarities with human extraintestinal pathogenic E. coli genomes. Journal of Bacteriology. DOI: 10.1128/JB.01726-06.

Kariyawasam S, Wilkie BN and Gyles CL (2004) Construction, characterization, and evaluation of the vaccine potential of three genetically defined mutants of avian pathogenic Escherichia coli. Avian Diseases. DOI: 10.1637/7093.

La Ragione RM, Woodward MJ, Kumar M, et al. (2013) Efficacy of a live attenuated Escherichia coli O78:K80 vaccine in chickens and Turkeys. Avian Diseases. DOI: 10.1637/10326-081512-Reg.1.

Landman WJM and van Eck JHH (2017) The efficacy of inactivated Escherichia coli autogenous vaccines against the E. coli peritonitis syndrome in layers. Avian Pathology. DOI: 10.1080/03079457.2017.1346231.

Li L, Thøfner I, Christensen JP, et al. (2017) Evaluation of the efficacy of an autogenous Escherichia coli vaccine in broiler breeders. Avian Pathology. DOI: 10.1080/03079457.2016.1267857.

Melamed D, Leitner G and Heller ED (1991) A vaccine against avian colibacillosis based on ultrasonic inactivation of Escherichia coli. Avian diseases. DOI: 10.2307/1591289.

Mohamed MA, Bakhit BM, Ibrahim AA, et al. (2016) Evaluation of the living Escherichia coli-O78 deleted aroA vaccine against homologous and heterologous E. coli challenge in broiler chickens. Journal of Advanced Veterinary Research 6(3): 89-92.

Nhung NT, Chansiripornchai N and Carrique-Mas JJ (2017) Antimicrobial resistance in bacterial poultry pathogens: A review. Frontiers in Veterinary Science. DOI: 10.3389/fvets.2017.00126.

Nolan LK, Vaillancourt J, Barbieri NL, et al. (2020) Colibacillosis. In: Diseases of Poultry. DOI: 10.1002/9781119371199.ch18.

Rodriguez-Siek KE, Giddings CW, Doetkott C, et al. (2005) Comparison of Escherichia coli isolates implicated in human urinary tract infection and avian colibacillosis. Microbiology. DOI: 10.1099/mic.0.27499-0.

Roth N, Käsbohrer A, Mayrhofer S, et al. (2019) The application of antibiotics in broiler production and the resulting antibiotic resistance in Escherichia coli: A global overview. Poultry Science. DOI: 10.3382/ps/pey539.

Swelum AA, Elbestawy AR, El-Saadony MT, et al. (2021) Ways to minimize bacterial infections, with special reference to Escherichia coli, to cope with the first-week mortality in chicks: an updated overview. Poultry Science. DOI: 10.1016/j.psj.2021.101039.

**Claims**

1. A composition comprising an immunologically effective amount of cells of *Escherichia coli* (*E. coli*) species killed by irradiation in a pharmaceutically acceptable carrier, wherein said killed cells are produced by exposing whole, intact cells of an *E. coli* species to irradiation under conditions effective to kill said cells so as to obtain a complete loss of multiplication ability in the cells while retaining the ability to elicit a protective immune response in an animal against the *E. coli* species, wherein the ability is **characterised by** a metabolic activity of the composition comprising the irradiated E. coli species of at least 25 %, preferably at least 35 %, especially at least 50 %, in a resazurin-based test for metabolic activity, wherein metabolic activity is measured by the percentage of reduction of resazurin (7-hydroxy-3H-phenoxazin-3-one 10-oxide) to resorufin (7-hydroxy-3H-phenoxa-zin-3-one).

2. The composition of claim 1, wherein the complete loss of multiplication ability of the killed cells is determined by:

   (i) streaking suspensions of the irradiated cells on MacConkey agar plates and enrich aliquots in LB (Luria-Bertani) broth in parallel,
   (ii) incubating both, agar plates and broth at 37°C for overnight,
   (iii) streaking the culture in LB broth the next day on MacConkey agar plates and incubating agar plates at 37°C for overnight, and
   (iv) detecting no growth on agar plates before and after in vitro enrichment.

3. The composition of claim 1 or 2, wherein the composition is suitable for aerosol delivery by comprising aerosol particles with an average diameter of 20 $\mu$m or lower, preferably of 1 to 20 $\mu$m, especially of 2 to 10 $\mu$m.

4. The composition of any one of claims 1 to 3, further comprising an adjuvant, preferably Freund's incomplete adjuvant, Freund's complete adjuvant, alum, microparticles, nanoparticles, beads, and oil.

5. The composition of any one of claims 1 to 4, wherein the resazurin-based test for metabolic activity is performed by using about $1 \times 10^8$ killed cells in a volume of 100 μl and incubating the cells and resazurin in a concentration of from 40 to 50 μM at a pH of from 7.0 to 7.5 for three hours at 37°, and then measuring optical densities (ODs), preferably with an enzyme linked immunosorbent assay (ELISA) reader, at wavelengths of 600 and 550 nm and determining the percentage reduction of resazurin according to the following equation:

$$\text{Percentage reduction of resazurin} = [A_{LW} - (A_{HW} \times R_O)] \times 100;$$

$A_{LW}$ =absorbance at lower wavelength minus the media blank
$A_{HW}$ =absorbance at higher wavelength minus the media blank
$R_O$ = correction factor (0.50466).

6. The composition of any one of claims 1 to 5, wherein the metabolic activity of the composition comprising the irradiated E. coli species is at least 60 %, preferably at least 70 %, especially at least 80 %, in the resazurin-based test for metabolic activity.

7. The composition of any one of claims 1 to 5, comprising a dose of killed cells of $10^6$ to $10^{11}$ cells, preferably of $10^7$ to $10^{10}$ cells, especially of $1,0 \times 10^8$ to $1.5 \times 10^9$ cells.

8. The composition of any one of claims 1 to 7, wherein the killed cells are PA14/17480/5-ovary, PA15/19103-3, or mixtures thereof, preferably PA14/17480/5-ovary.

9. The composition of any one of claims 1 to 8, wherein the cells are cells of an avian pathogenic *E. coli* (APEC) strain or mixtures of APEC strains.

10. The composition of any one of claims 1 to 7, wherein the killed cells are contained as a suspension having an approximate concentration between $10^6$ to $10^{11}$, preferably of $10^6$ to $10^{10}$, preferably of $10^7$ to $10^9$ CFU/ml

11. Composition according to any one of claims 1 to 10 for use in a method for controlling an *E. coli* species in an animal comprising administering to the animal the composition to elicit a protective immune response in an animal against said *E. coli* species.

12. Composition for use of claim 11, wherein the animal is poultry selected from the group consisting of chickens, turkeys, ducks, quail, and geese, preferably wherein the animal is an animal being younger than four weeks, especially younger than two weeks.

13. Composition for use of claim 11 or 12, wherein the composition is administered as an aerosol to the animal.

14. Composition for use of any one of claims 11 to 13, wherein the animal is chicken with an age of not more than 10 days, preferably with an age of not more than 7 days, especially of not more than 4 days.

Fig. 1A

Fig. 1B

Fig. 1C

Fig. 2A

Fig. 2B

Fig. 3

Fig. 4A

Fig. 4B

Fig. 4C

Fig. 4D

Fig. 4E

Fig. 5

Fig. 6

Fig. 7

Fig. 8

Fig. 9

Fig. 10

EP 4 252 770 A1

Fig. 11

Fig. 12

EP 4 252 770 A1

Fig. 13

Fig. 14

Fig. 15A

Fig. 15B

Fig. 16

Fig. 17

Fig. 18

Fig. 19

Europäisches
Patentamt
European
Patent Office
Office européen
des brevets

## EUROPEAN SEARCH REPORT

**Application Number**

EP 22 16 6276

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MELAMED D ET AL:  "A VACCINE AGAINST AVIAN COLIBACILLOSIS BASED ON ULTRASONIC INACTIVATION OF ESCHERICHIA COLI", AVIAN DISEASES, AMERICAN ASSOCIATION OF AVIAN PATHOLOGISTS, KENNET SQ., PA, US, vol. 35, no. 1, 1 January 1991 (1991-01-01), pages 17-22, XP009038504, ISSN: 0005-2086 * figure 1 * * material and methods:; page 18, column 1 - column 2, paragraph 6 * * page 19, column 1, last paragraph - column 2, paragraph 1 * ----- | 1-7, 9-12,14 | INV. A61K39/108 A61P31/04 |
| X | Dima Vf:  "Development of an irradiated vaccine that protects against enterotoxigenic Escherichia coli diarrhoea", , 1 January 1992 (1992-01-01), XP055967014, Retrieved from the Internet: URL:http://www.ncbi.nlm.nih.gov/pubmed/1457822 [retrieved on 2022-09-30] * page 6, paragraph 2-7 * * page 6, last paragraph - page 7, paragraph 2 * * page 7, last paragraph - page 8, paragraph 1; table 1 * ----- -/-- | 1-3,5-7, 10,11 | TECHNICAL FIELDS SEARCHED (IPC) A61K A61P |

The present search report has been drawn up for all claims

| Place of search | Date of completion of the search | Examiner |
|---|---|---|
| The Hague | 3 October 2022 | Noë, Veerle |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**page 1 of 2**

Europäisches
Patentamt

European
Patent Office

Office européen
des brevets

# EUROPEAN SEARCH REPORT

Application Number

EP 22 16 6276

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JALILI ZAHRA ET AL: "Characterization of killed but metabolically active uropathogenicEscherichia colistrain as possible vaccine candidate for urinary tract infection", MICROBIAL PATHOGENESIS, ACADEMIC PRESS LIMITED, NEW YORK, NY, US, vol. 122, 20 June 2018 (2018-06-20), pages 184-190, XP085447849, ISSN: 0882-4010, DOI: 10.1016/J.MICPATH.2018.06.027 * abstract * * page 185, paragraph 2.3.4 * * page 186, paragraph 3.4 * * page 188, column 2, paragraph 2-3 * ----- | 1,2,6,11 | |
| A | BROCKSTEDT D G ET AL: "Killed but metabolically active microbes: a new vaccine paradigm for eliciting effector T-cell responses and protective immunity", NATURE MEDICINE, NATURE PUBLISHING GROUP US, NEW YORK, vol. 11, no. 8, 24 July 2005 (2005-07-24), pages 853-860, XP037135405, ISSN: 1078-8956, DOI: 10.1038/NM1276 [retrieved on 2005-07-24] * the whole document * ----- | 1-14 | TECHNICAL FIELDS SEARCHED (IPC) |

The present search report has been drawn up for all claims

| Place of search | Date of completion of the search | Examiner |
|---|---|---|
| The Hague | 3 October 2022 | Noë, Veerle |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
........................................................................
& : member of the same patent family, corresponding
document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8173139 B1
- WO 2004103402 A1

**Non-patent literature cited in the description**

- **KOUTSIANOS et al.** *Vet. Sci.,* 2020, vol. 7, 80
- **HAGGERTY et al.** *J. Geophys. Res.,* 2009, vol. 114, 942
- **COSTA et al.** *Antibiotics,* 2021, vol. 19, 974
- **REZAEE et al.** *Vet. Pathol.,* 2021, vol. 58, 71-79
- **GAUßMANN et al.** *Avian Pathol,* 2018, vol. 47, 271-280
- **BAGHERI et al.** *Frontiers,* 2022, 864656
- **KATHAYAT et al.** *Pathogens,* 2021, vol. 10, 0046
- **MEHAT et al.** *Av. Pathol.,* 2021, vol. 50, 402-416
- **THOMRONGSUWANNAKIJ et al.** *Poultry Sci,* 2022, vol. 101, 101527
- **NEWMAN et al.** *PeerJ,* vol. 9 (20121), e1102
- **ABD EL-MAWGOUD AI ; EL-NAHASS ES ; SHANY SAS et al.** Efficacy of live attenuated vaccine and commercially available lectin against avian pathogenic E. coli infection in broiler chickens. *VETERINARY SCIENCES,* 2020
- **ALBER A ; STEVENS MP ; VERVELDE L.** The bird's immune response to avian pathogenic Escherichia coli. *Avian Pathology,* 2021
- **CORBANIE EA ; MATTHIJS MGR ; VAN ECK JHH et al.** Deposition of differently sized airborne microspheres in the respiratory tract of chickens. *Avian Pathology,* 2006
- **FERTEY.** *Viruses,* 2016, vol. 8, 319
- **FILHO TF ; FÁVARO C ; INGBERMAN M et al.** Effect of spray escherichia coli vaccine on the immunity of poultry. *Avian Diseases,* 2013
- **GHARIB A ; HAMOUDA A ; BDEL-WAHAB AA et al.** Protective Efficacy of a Commercial Live Attenuated aroA mutant Vaccine Against Avian Pathogenic Escherichia coli Challenge in Broilers. *Zagazig Veterinary Journal,* 2017
- **GHUNAIM H ; ABU-MADI MA ; KARIYAWASAM S.** Advances in vaccination against avian pathogenic Escherichia coli respiratory disease: Potentials and limitations. *Veterinary Microbiology,* 2014
- **GREGERSEN RH ; CHRISTENSEN H ; EWERS C et al.** Impact of Escherichia coli vaccine on parent stock mortality, first week mortality of broilers and population diversity of E. coli in vaccinated flocks. *Avian Pathology,* 2010
- **HELLFRITZSCH M ; SCHERLIEB R.** Mucosal vaccination via the respiratory tract. *Pharmaceutics,* 2019
- **JOHNSON TJ ; KARIYAWASAM S ; WANNEMUEHLER Y et al.** The genome sequence of avian pathogenic Escherichia coli strain O1:K1:H7 shares strong similarities with human extraintestinal pathogenic E. coli genomes. *Journal of Bacteriology,* 2007
- **KARIYAWASAM S ; WILKIE BN ; GYLES CL.** Construction, characterization, and evaluation of the vaccine potential of three genetically defined mutants of avian pathogenic Escherichia coli. *Avian Diseases,* 2004
- **LA RAGIONE RM ; WOODWARD MJ ; KUMAR M et al.** Efficacy of a live attenuated Escherichia coli O78:K80 vaccine in chickens and Turkeys. *Avian Diseases,* 2013
- **LANDMAN WJM ; VAN ECK JHH.** The efficacy of inactivated Escherichia coli autogenous vaccines against the E. coli peritonitis syndrome in layers. *Avian Pathology,* 2017
- **LI L ; THØFNER I ; CHRISTENSEN JP et al.** Evaluation of the efficacy of an autogenous Escherichia coli vaccine in broiler breeders. *Avian Pathology,* 2017
- **MELAMED D ; LEITNER G ; HELLER ED.** A vaccine against avian colibacillosis based on ultrasonic inactivation of Escherichia coli. *Avian diseases,* 1991
- **MOHAMED MA ; BAKHIT BM ; IBRAHIM AA et al.** Evaluation of the living Escherichia coli-O78 deleted aroA vaccine against homologous and heterologous E. coli challenge in broiler chickens. *Journal of Advanced Veterinary Research,* 2016, vol. 6 (3), 89-92

- **NHUNG NT ; CHANSIRIPORNCHAI N ; CARRIQUE-MAS JJ.** Antimicrobial resistance in bacterial poultry pathogens: A review. *Frontiers in Veterinary Science,* 2017
- **NOLAN LK ; VAILLANCOURT J ; BARBIERI NL et al.** Colibacillosis. *Diseases of Poultry,* 2020
- **RODRIGUEZ-SIEK KE ; GIDDINGS CW ; DOETKOTT C et al.** Comparison of Escherichia coli isolates implicated in human urinary tract infection and avian colibacillosis. *Microbiology,* 2005
- **ROTH N ; KÄSBOHRER A ; MAYRHOFER S et al.** The application of antibiotics in broiler production and the resulting antibiotic resistance in Escherichia coli: A global overview. *Poultry Science,* 2019
- **SWELUM AA ; ELBESTAWY AR ; EL-SAADONY MT et al.** Ways to minimize bacterial infections, with special reference to Escherichia coli, to cope with the first-week mortality in chicks: an updated overview. *Poultry Science,* 2021